# EUROPÄISCHE PATENTANMELDUNG

(11) **EP 1 184 391 A2**
(43) Veröffentlichungstag der Anmeldung: **06.03.2002**
(21) Anmeldenummer: 01119704.3
(22) Anmeldetag: 24.08.2001
(51) Int. Cl.: C07K 14/705, C12N 15/11, C12P 19/34, G01N 33/68, C12N 15/63

(54) **Acetylcholinrezeptor-Untereinheiten**

(30) Priorität: 28.08.2000 DE 10042177
(71) Anmelder: BAYER AG, 51368 Leverkusen (DE)
(72) Erfinder: Adamczewski, Martin, Dr., 51067 Köln (DE); Ebbinghaus-Kintscher, Ulrich, Dr., 44287 Dortmund (DE); Methfessel, Christoph, Dr., 42327 Wuppertal (DE); Schulte, Thomas, Dr., 51061 Köln (DE)

(57) **Zusammenfassung**

Die Erfindung betrifft modifizierte Acetylcholinrezeptor-Untereinheiten, dafür codierende Nukleinsäuren, sowie ein Verfahren zum Auffinden von Wirkstoffen für den Pflanzenschutz bzw. pharmazeutischen Wirkstoffen für die Behandlung von Mensch und/oder Tier.

## Beschreibung

Die Erfindung betrifft modifizierte Acetylcholinrezeptor-Untereinheiten, dafür codierende Nukleinsäuren, sowie ein Verfahren zum Auffinden von Wirkstoffen für den Pflanzenschutz bzw. pharmazeutischen Wirkstoffen für die Behandlung von Mensch und/oder Tier.

Nikotinische Acetylcholinrezeptoren sind ligandengesteuerte lonenkanäle, die eine Rolle bei der Neurotransmission im Tierreich spielen. Die Bindung von Acetylcholin oder anderen Agonisten an den Rezeptor verursacht eine vorübergehende Öffnung des Kanals und gestattet den Durchstrom von Kationen. Man nimmt an, dass ein Rezeptor aus fünf Untereinheiten besteht, die sich um eine Pore gruppieren. Jede dieser Untereinheiten ist ein Protein, das aus einem extrazellulären N-terminalen Teil besteht, gefolgt von drei Transmembranregionen, einem intrazellulären Teil, sowie einer vierten Transmembranregion und einem kurzen extrazellulären C-terminalen Teil. Bestimmte Untereinheiten tragen auf ihrem extrazellulären Teil die Bindestelle für Liganden wie Acetylcholin. Zwei vicinale Cysteine sind Bestandteil dieser Bindestelle und daher gemeinsames Strukturmerkmal aller ligandenbindenden Untereinheiten, die auch als α-Untereinheiten bezeichnet werden. Untereinheiten ohne dieses Strukturmerkmal werden je nach Lokalisation und Funktion des Rezeptors als β-, γ-, δ- oder ε-Untereinheiten bezeichnet (Changeux et al. 1992).

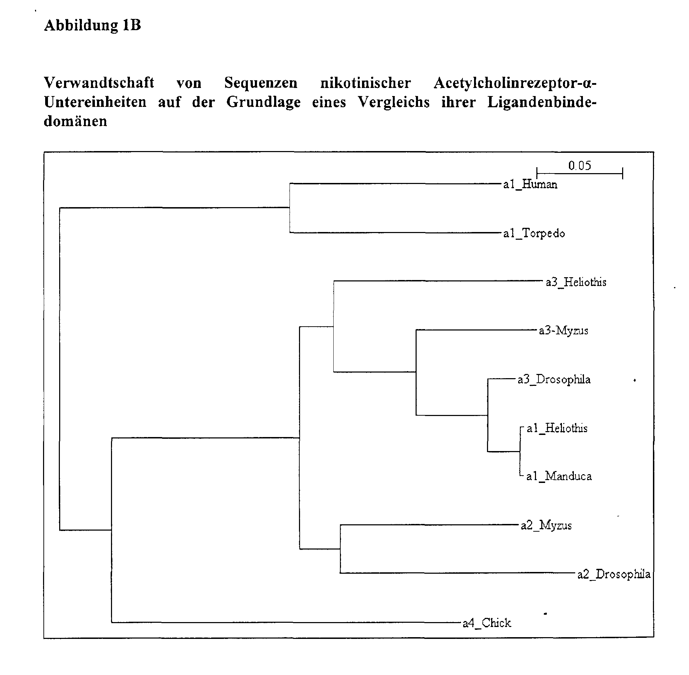

Acetylcholinrezeptoren sind vor allem in Wirbeltieren gut untersucht. Anhand ihrer anatomischen Lokalisierung und ihrer funktionellen Eigenschaften (Leitungseigenschaften des Kanals, Desensibilisierung, Sensitivität gegenüber Agonisten und Antagonisten sowie gegenüber Toxinen wie z.B. α-Bungarotoxin) lassen sich hier drei Gruppen unterscheiden. Die Einteilung korreliert mit der molekularen Zusammensetzung der Rezeptoren. Es gibt heterooligomere Rezeptoren mit der Untereinheitenzusammensetzung α₂βγδ, die im Muskel vorkommen (Noda et al. 1982, Claudio et al. 1983, Devillers-Thiery et al. 1983, Noda et al. 1983a, b), heterooligomere Rezeptoren, die Untereinheiten aus der Gruppe α2 - α6 und β2 - β4 enthalten, und die im Nervensystem vorkommen (Schoepfer et al. 1990, Heinemann et al. 1997) sowie homooligomere Rezeptoren, die Untereinheiten aus der Gruppe α7 - α9 enthalten, und die ebenfalls im Nervensystem vorkommen (Lindstrom et al. 1997, Elgoyhen et al. 1997). Diese Einteilung wird auch durch die Verwandschaft der Gensequenzen der verschiedenen Untereinheiten gestützt. Typischerweise sind die Sequenzen funktionell homologer Untereinheiten verschiedener Spezies ähnlicher als Sequenzen von Untereinheiten aus verschiedenen Gruppen, aber der gleichen Spezies. Dies ist mit einigen Beispielen in der Abbildung 1B dargestellt. Weiterhin sind die Gensequenzen aller bekannten Acetylcholinrezeptor-Untereinheiten nicht nur untereinander in gewissem Maße ähnlich, sondern auch mit denen einiger anderer ligandengesteuerter Ionenkanäle (z.B. den Serotoninrezeptoren vom Typ 5HT₃, den GABA-gesteuerten Chloridkanälen, den Glycin-gesteuerten Chloridkanälen). Man geht daher davon aus, dass alle diese Rezeptoren von einem gemeinsamen Vorläufer abstammen und ordnet sie in eine Gen-Superfamilie ein (Ortells et al. 1995).

In Insekten ist Acetylcholin der wichtigste exzitatorische Neurotransmitter des zentralen Nervensystems. Dementsprechend lassen sich Acetylcholinrezeptoren an Präparaten zentraler Ganglien aus Insekten elektrophysiologisch nachweisen. Der Nachweis gelingt sowohl an post- als auch an präsynaptischen Nervenendigungen sowie an den Zellkörpern von Interneuronen, Motorneuronen und modulatorischen Neuronen (Breer et al. 1987, Buckingham et al. 1997). Unter den Rezeptoren gibt es solche, die durch α-Bungarotoxin inhibiert werden, und solche, die insensitiv sind (Schloß et al. 1988). Die Acetylcholinrezeptoren sind außerdem der molekulare Angriffspunkt wichtiger natürlicher (z.B. Nikotin) und synthetischer Insektizide (z.B. Chloronikotinyle).

Die Gensequenzen einer Anzahl von nikotinischen Acetylcholinrezeptoren der Insekten sind bereits bekannt. So sind in Drosophila melanogaster die Sequenzen fünf verschiedener Untereinheiten beschrieben (Bossy et al. 1988, Hermanns-Borgmeyer et al. 1986, Sawruk et al. 1990a, 1990b, Schulz et al. 1998), in Locusta migratoria ebenfalls fünf (Hermsen et al. 1998), in Schistocerca gregaria eine (Marshall et al. 1990), in Myzus persicae sechs (Sgard et al. 1998, Huang et al. 1999), in Manduca sexta zwei Sequenzen (Eastham et al. 1997, Genbank AJ007397) und in Heliothis virescens sechs (Genbank AF 096878, AF 096879, AF 096880, AF143846, AF143847, AJ 000399). Zudem ist eine Reihe von partiellen Gensequenzen aus Drosophila melanogaster als sog. expressed sequence tags charakterisiert worden (Genbank AA540687, AA698155, AA697710, AA697326). Alle diese Sequenzen werden in α- und β-Untereinheiten klassifiziert, je nachdem, ob die zwei vicinalen Cysteine der Ligandenbindestelle vorhanden sind oder nicht.

Die rekombinante Expression nikotinischer Rezeptoren aus Insekten hat sich als schwieriger erwiesen als die der analogen Rezeptoren aus Vertebraten oder C. elegans. So ist es bisher nicht gelungen, nikotinische Rezeptoren, die nur aus Untereinheiten von Insekten bestehen, so zu exprimieren, dass ihre funktionalen Eigenschaften denen natürlicher Rezeptoren gleichen (Marshall et al. 1990, Amar et al. 1995, Hermsen et al. 1998, Sgard et al. 1998). Relevante funktionale Eigenschaften sind z.B. die Empfindlichkeit gegen Agonisten und Antagonisten, die Leitfähigkeit für Ionenströme oder die Desensitivierung. Zumindest einige α-Untereinheiten aus verschiedenen Insektenspezies tragen jedoch zu einem funktionellen Rezeptor bei, wenn statt einer Insekten-β-Untereinheit eine nicht-α-Untereinheit aus Vertebraten koexprimiert wird. In Oocyten von Xenopus laevis ist die ligandeninduzierte Leitfähigkeit solcher hybrider Rezeptoren untersucht worden. Kombinationen z.B. der Drosophila α1-, α2- oder α3-Untereinheit mit der β2-Untereinheit von Huhn oder Ratte führen zu Rezeptoren, die in ihrer Empfindlichkeit gegen Agonisten und Antagonisten oder ihrer Leitfähigkeit für Ionenströme solchen Rezeptoren ähneln, die in Nativpräparaten nachgewiesen werden (Bertrand et al. 1994, Lansdell et al. 1997, Schulz et al. 1998, 2000, Matsuda et al. 1998). Dagegen konnte in Zellinien die Expression hybrider Rezeptoren, die z.B. aus Kombinationen der Myzus persicae α1-, α2- oder α3-Untereinheit mit der β2-Untereinheit der Ratte oder aus Kombinationen der Drosophila α1-, α2- oder α3-Untereinheit mit der β2- oder β4-Untereinheit der Ratte bestehen, bisher nur durch die Bindung nikotinischer Liganden (Lansdell et al. 1997, 2000, Huang et al. 1999) nachgewiesen werden. Die ligandeninduzierte Leitfähigkeit solcher Rezeptoren wurde bisher in keinem einzigen Fall nachgewiesen.

Einen weiteren Versuch der Annäherung an die Expression nikotinischer Rezeptoren aus Insekten stellen chimäre Untereinheiten dar (van den Beukel 1998). In die Gensequenz der α7-Untereinheit der Ratte wurden gentechnisch Abschnitte der Gensequenz der α2-Untereinheit aus Drosophila eingefügt. Die Expression der Chimären in Oocyten von Xenopus laevis konnte durch Bindung nikotinischer Liganden nachgewiesen werden, aber auch diese Rezeptoren wiesen keine ligandeninduzierte Leitfähigkeit auf.

Die rekombinante Ausprägung nikotinischer Rezeptoren aus Insekten oder solcher nikotinischer Rezeptor-Konstrukte, die den Rezeptoren aus Insekten in ihrer Empfindlichkeit gegen Agonisten und Antagonisten und in ihrer ligandeninduzierten Leitfähigkeit für Ionenströme entsprechen, in eukaryotischen Zelllinien ist nicht nur ein bisher ungelöstes wissenschaftliches Problem, sondern ist auch von großer praktischer Bedeutung, beispielsweise für die Etablierung von Testsystemen mit hohem Durchsatz für die Suche nach neuen Wirkstoffen für den Pflanzenschutz bzw. pharmazeutischen Wirkstoffe für die Behandlung von Mensch und/oder Tier.

Der vorliegenden Erfindung liegt somit insbesondere die Aufgabe zu Grunde, ein Testverfahren bzw. die Bestandteile eines Testverfahrens zur Verfügung zu stellen, mit dem Verbindungen aufgefunden werden können, welche als Modulatoren, insbesondere als Agonisten oder Antagonisten, die Leitungseigenschaften nikotinischer Rezeptoren aus Insekten verändern. Solche Verbindungen können als Wirkstoffe für den Pflanzenschutz bzw. pharmazeutische Wirkstoffe für die Behandlung von Mensch und/oder Tier genutzt werden.

Die Aufgabe wird gelöst durch die Bereitstellung von modifizierten Acetylcholinrezeptor-Untereinheiten, wobei zumindest eine Aminosäure im Bereich einer α-Untereinheit eines Vertebraten-Acetylcholinrezeptors, welcher zu der Aminosäuresequenz gemäß SEQ ID NO: 1 homolog ist, durch eine Aminosäure, die an der identischen Position in dem entsprechenden Bereich einer α-Untereinheit eines Insekten-Acetylcholinrezeptors vorkommt, ersetzt ist. Der Austausch muss natürlich zu einer Änderung der entsprechenden Aminosäuresequenz im Vergleich mit der Ursprungssequenz führen.

Die Homologie bzw. Ähnlichkeit von Bereichen und die Konkordanz der Aminosäurepositionen zweier oder mehrerer Rezeptor-Untereinheiten kann durch Aminosäuresequenz-Vergleiche mit üblichen Methoden festgestellt werden. Eine übliche Methode beinhaltet die Verwendung der Programme "Gap" bzw. "Pileup" aus dem Programmpaket GCG Version 10.0 (GCG Genetics Computer Group, Inc., Madison Wisconsin, USA) zum Vergleich zweier bzw. mehrerer Aminosäuresequenzen. Es können auch das ClustalX Programm (Version 1.81) (Thompson et al. 1997, IGBMC, Straßburg, Frankreich) oder andere ähnliche Programme verwendet werden. Die Programme werden mit Standardeinstellungen verwendet. Die zu vergleichenden Sequenzen umfassen den Bereich vom N-Terminus des Proteins bis zur ersten Transmembranregion.

Als "an der identischen Position in zwei oder mehreren Rezeptor-Untereinheiten vorkommend" gelten solche Aminosäuren, die von den Sequenzvergleichsprogrammen in den entsprechenden Sequenzen der verglichenen Untereinheiten untereinander angeordnet werden. Als "homologer Bereich" zweier oder mehrerer Rezeptor-Untereinheiten wird ebenso ein Aminosäuresequenzbereich angesehen, der von den Sequenzvergleichsprogrammen untereinander angeordnet wird.

Wegen der unterschiedlichen Nummerierung strukturell und/oder funktionell entsprechender Bereiche in Acetylcholinrezeptor-Untereinheiten verschiedener Spezies wird die α-Untereinheit des Rochens Torpedo califomica als Bezugsmaßstab für die Beschreibung der auszutauschenden Aminosäure(n) oder Bereiche verwendet. Die Aminosäuresequenz gemäß SEQ ID NO: 1 entspricht demjenigen Bereich der α-Untereinheit von Torpedo californica, der mit der Aminosäure, die mit 123 gekennzeichnet ist, beginnt und mit der Aminosäure, die mit 167 gekennzeichnet ist, endet. Die Numerierung wurde übernommen aus dem Eintrag "Acetylcholine Receptor Protein, Alpha Chain Precursor" der Swissprot-Datenbank (P02710).

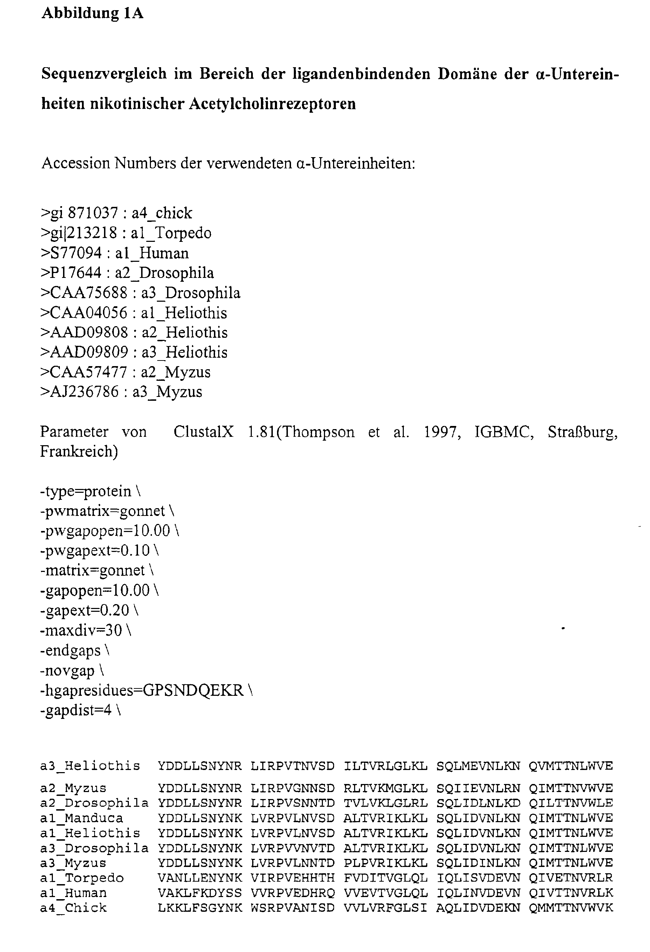

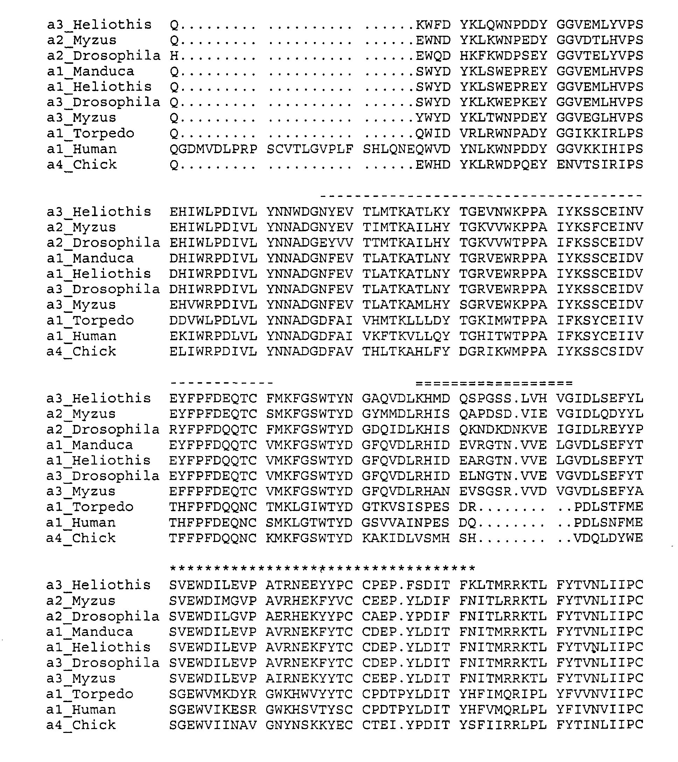

Die Abbildung 1A zeigt als Ergebnis eines Sequenzvergleichs die Konkordanz der Aminosäuren und die Homologie der Bereiche auf der Grundlage von Aminosäuresequenzen verschiedener, relevanter Untereinheiten. Die Abbildung 1B stellt dar, dass es durch Sequenzvergleich möglich ist, die Untereinheiten gemäß ihrer Funktion und Lokalisierung zu gruppieren und zwischen Insekten- und Vertebraten-Rezeptor-Untereinheiten zu differenzieren. Für diese Analyse wurde das njplotwin95-Programm aus dem WWW-Query-Programmpaket (Perrière et al. 1996) verwendet. Diese Analyse kann ohne Weiteres auf andere, sogar auf bisher unbekannte Acetylcholinrezeptor-Untereinheiten erstreckt werden.

Bevorzugt sind in den erfindungsgemäßen Acetylcholinrezeptor-Untereinheiten mindestens vier, besonders bevorzugt mindestens sieben, ganz besonders bevorzugt alle Aminosäuren in dem oben beschriebenen Bereich einer α-Untereinheit eines Vertebraten-Acetylcholinrezeptors durch die entsprechende Anzahl von Aminosäuren einer α-Untereinheit eines Insekten-Acetylcholinrezeptors ersetzt.

Solche modifizierten Untereinheiten weisen eine höhere Sensitivität für insektizide Wirkstoffe, wie z.B. Imidacloprid, auf als eine nicht-modifizierte Untereinheit.

Bevorzugt handelt es sich bei den oben erwähnten α-Untereinheiten von Acetylcholinrezeptoren aus Vertebraten um neuronale Untereinheiten von Maus, Ratte, Huhn, Hund, Zebrafisch, Rhesusaffe, Rind oder Schwein.

Bevorzugt handelt es sich bei den oben erwähnten α-Untereinheiten von Acetylcholinrezeptoren aus Insekten um die α2-Untereinheit oder die α3-Untereinheit von Myzus persicae, oder um die α1-Untereinheit von Heliothis virescens oder Manduca sexta, oder um die α1-, α2- oder α3-Untereinheit von Drosophila melanogaster.

Besonders bevorzugt ist eine modifizierte Acetylcholinrezeptor-Untereinheit mit einer Aminosäuresequenz gemäß SEQ ID NO: 3.

Gegenstand der vorliegenden Erfindung sind auch Acetylcholinrezeptoren, welche die erfindungsgemäßen Untereinheiten umfassen. Als strukturelle Partner der erfindungsgemäßen Untereinheiten enthalten diese Rezeptoren bevorzugt eine β2-Untereinheit von Maus, Ratte, Huhn, Hund, Zebrafisch, Rhesusaffe, Rind oder Schwein.

Auch die nicht-modifizierten Bereiche der erfindungsgemäßen Untereinheiten müssen nicht identisch mit den entsprechenden Bereichen natürlich vorkommender α-Untereinheiten von Vertebraten-Acetylcholinrezeptoren sein, solange gewährleistet ist, dass die Rezeptoren eine ligandeninduzierte Leitfähigkeit für Ionenströme aufweisen.

Solche Unterschiede können an verschiedenen Stellen und mehrfach in einer α-Untereinheit vorkommen, wie beispielsweise an dem Peptid-Rückgrat, an der Aminosäure-Seitenkette, am Amino- und/oder am Carboxy-Terminus. Sie umfassen beispielsweise Acetylierungen, Acylierungen, ADP-Ribosylierungen, Amidierungen, kovalente Verknüpfungen mit Flavinen, Häm-Anteilen, Nukleotiden oder Nukleotid-Derivaten, Lipiden oder Lipid-Derivaten oder Phosphatidylinositol, Cyclisierungen, Disulfidbrückenbildungen, Demethylierungen, Cystin-Bildungen, Formylierungen, gamma-Carboxylierungen, Glycosylierungen, Hydroxylierungen, Iodierungen, Methylierungen, Myristoylierungen, Oxidationen, proteolytische Prozessierungen, Phosphorylierungen, Selenoylierungen und tRNA-vermittelte Additionen von Aminosäuren.

Weiterhin können die erfindungsgemäßen Untereinheiten im Vergleich zu den entsprechenden Bereichen natürlich vorkommender Acetylcholinrezeptor-Untereinheiten Deletionen oder Aminosäuresubstitutionen aufweisen, solange sie noch die oben erwähnte Leitfähigkeit vermitteln können. Konservative Substitutionen sind bevorzugt. Solche konservativen Substitutionen umfassen Variationen, wobei eine Aminosäure durch eine andere Aminosäure aus der folgenden Gruppe ersetzt wird:
1. Kleine aliphatische, nicht-polare oder wenig polare Reste: Ala, Ser, Thr, Pro und Gly;
2. Polare, negativ geladene Reste und deren Amide: Asp, Asn, Glu und Gln;
3. Polare, positiv geladene Reste: His, Arg und Lys;
4. Große aliphatische, nicht-polare Reste: Met, Leu, Ile, Val und Cys; und
5. Aromatische Reste: Phe, Tyr und Trp.

Die folgende Liste zeigt bevorzugte konservative Substitutionen:

| **Ursprünglicher Rest** | **Substitution** |
|---|---|
| Ala | Gly, Ser |
| Arg | Lys |
| Asn | Gin, His |
| Asp | Glu |
| Cys | Ser |
| Gln | Asn |
| Glu | Asp |
| Gly | Ala, Pro |
| His | Asn, Gln |
| Ile | Leu, Val |
| Leu | Ile, Val |
| Lys | Arg, Gln, Glu |
| Met | Leu, Tyr, Ile |
| Phe | Met, Leu, Tyr |
| Ser | Thr |
| Thr | Ser |
| Trp | Tyr |
| Tyr | Trp, Phe |
| Val | Ile, Leu |

Gegenstand der vorliegenden Erfindung sind auch Nukleinsäuren, die für die erfindungsgemäßen Untereinheiten codieren.

Bei den erfindungsgemäßen Nukleinsäuren handelt es sich insbesondere um einzelsträngige oder doppelsträngige Desoxyribonukleinsäuren (DNA) oder Ribonukleinsäuren (RNA). Bevorzugte Ausführungsformen sind Fragmente genomischer DNA, die Introns enthalten können, und cDNAs.

Eine bevorzugte Ausführungsform der erfindungsgemäßen Nukleinsäuren stellt eine cDNA dar, welche die Nukleotidsequenz gemäß SEQ ID NO: 2 besitzt.

Gegenstand der vorliegenden Erfindung sind weiterhin DNA-Konstrukte, die eine erfindungsgemäße Nukleinsäure und einen heterologen Promotor umfassen.

Der Ausdruck "heterologer Promotor", wie er hierin verwendet wird, bezieht sich auf einen Promotor, der andere Eigenschaften als derjenige Promotor aufweist, der im Ursprungsorganismus die Expression des betreffenden Gens kontrolliert. Der Ausdruck "Promotor", wie er hierin verwendet wird, bezieht sich allgemein auf Expressionskontrollsequenzen.

Die Auswahl von heterologen Promotoren ist davon abhängig, ob zur Expression pro- oder eukaryotische Zellen oder zellfreie Systeme verwendet werden. Beispiele für heterologe Promotoren sind der frühe oder späte Promotor des SV40, des Adenovirus oder des Cytomegalovirus, der "immediate early" Promotor aus Baculovirus, der Metallothionin-Promotor aus Drosophila melanogaster, das lac-System, das trp-System, die Haupt-Operator- und Promotorregionen des Phagen lambda, die Kontrollregionen des fd-Hüllproteins, der Promotor der 3-Phosphoglyceratkinase, der Promotor der Sauren Phosphatase und der Promotor des α-Mating-Faktors der Hefe.

Gegenstand der Erfindung sind weiterhin Vektoren, die eine erfindungsgemäße Nukleinsäure bzw. ein erfindungsgemäßes DNA-Konstrukt enthalten. Als Vektoren können alle in molekularbiologischen Laboratorien verwendete Plasmide, Phasmide, Cosmide, YACs oder künstliche Chromosomen verwendet werden.

Gegenstand der vorliegenden Erfindung sind auch Wirtszellen, die eine erfindungsgemäße Nukleinsäure, ein erfindungsgemäßes DNA-Konstrukt oder einen erfindungsgemäßen Vektor enthalten.

Als Wirtszellen eignen sich sowohl prokaryotische Zellen, wie Bakterien der Gattungen Bacillus, Pseudomonas, Streptomyces, Streptococcus, Staphylococcus, vorzugsweise E. coli, als auch eukaryotische Zellen, wie Hefen, Säuger-, Amphibien-, Insekten- oder Pflanzenzellen. Bevorzugte eukaryotische Wirtszellen sind HEK-293-, Schneider S2-, Spodoptera Sf9-, CHO-, COS1-, COS7-Zellen und Pflanzenzellen in Zellkultur.

Gegenstand der vorliegenden Erfindung sind weiterhin Verfahren zum Herstellen der erfindungsgemäßen Untereinheiten. Zur Herstellung der Untereinheiten, die von den erfindungsgemäßen Nukleinsäuren codiert werden, können Wirtszellen, die eine der erfindungsgemäßen Nukleinsäuren enthalten, unter geeigneten Bedingungen kultiviert werden. Dabei kann die zu exprimierende Nukleinsäure an die "Codon Usage" der Wirtszellen angepasst werden. Die gewünschten Untereinheiten können danach auf übliche Weise aus den Zellen oder dem Kulturmedium isoliert werden. Die Untereinheiten können auch in in vitro-Systemen hergestellt werden.

Ein schnelles Verfahren zum Isolieren der erfindungsgemäßen Untereinheiten, die von Wirtszellen unter Verwendung einer erfindungsgemäßen Nukleinsäure synthetisiert werden, beginnt mit der Expression eines Fusionsproteins, wobei der Fusionspartner auf einfache Weise affinitätsgereinigt werden kann. Der Fusionspartner kann beispielsweise Glutathion S-Transferase sein. Das Fusionsprotein kann dann an einer Glutathion-Affinitätssäule gereinigt werden. Der Fusionspartner kann durch partielle proteolytische Spaltung beispielsweise an Linkern zwischen dem Fusionspartner und der zu reinigenden erfindungsgemäßen Untereinheit abgetrennt werden. Der Linker kann so gestaltet werden, dass er Ziel-Aminosäuren, wie Arginin- und Lysin-Reste einschließt, die Stellen für eine Spaltung durch Trypsin definieren. Um solche Linker zu erzeugen, können Standard-Klonierungsverfahren unter Verwendung von Oligonukleotiden angewendet werden.

Weitere mögliche Reinigungsverfahren basieren auf präparativer Elektrophorese, FPLC, HPLC (z.B. unter Anwendung von Gelfiltrations-, Reversphasen- oder leicht hydrophoben Säulen), Gelfiltration, differentieller Präzipitation, Ionenaustausch-Chromatographie und Affinitätschromatographie.

Da Acetylcholinrezeptoren aus Membranproteinen aufgebaut sind, werden in den Reinigungsverfahren vorzugsweise Detergensextraktionen durchgeführt, beispielsweise unter Verwendung von Detergenzien, die die Sekundär- und Tertiärstrukturen der Polypeptide nicht oder nur wenig beeinflussen, wie nicht-ionische Detergenzien.

Die Reinigung der erfindungsgemäßen Untereinheiten kann die Isolierung von Membranen ausgehend von Wirtszellen, die die erfindungsgemäßen Nukleinsäuren exprimieren, umfassen. Vorzugsweise exprimieren solche Zellen die erfindungsgemäßen Polypeptide in einer ausreichenden Kopienanzahl, so dass die Menge der Polypeptide in einer Membranfraktion mindestens 10-fach höher ist als diejenige, die in vergleichbaren Membranen von Zellen gefunden wird, die Acetylcholinrezeptoren natürlicherweise exprimieren; besonders bevorzugt ist die Menge mindestens 100-fach, ganz besonders bevorzugt mindestens 1000-fach höher.

Die Ausdrücke "Isolierung oder Reinigung", wie sie hierin verwendet werden, bedeuten, dass die erfindungsgemäßen Untereinheiten von anderen Proteinen oder anderen Makromolekülen der Zelle oder des Gewebes abgetrennt werden. Vorzugsweise ist eine die erfindungsgemäßen Untereinheiten enthaltende Zusammensetzung hinsichtlich des Proteingehalts gegenüber einer Präparation aus den Wirtszellen mindestens 10-fach und besonders bevorzugt mindestens 100-fach angereichert.

Die erfindungsgemäßen Untereinheiten können auch ohne Fusionspartner mit Hilfe von Antikörpern, die an die Polypeptide binden, affinitätsgereinigt werden.

Ferner sind auch Verfahren zum Herstellen der erfindungsgemäßen Nukleinsäuren Gegenstand der vorliegenden Erfindung. Die erfindungsgemäßen Nukleinsäuren können auf die übliche Weise hergestellt werden. Beispielsweise können die Nukleinsäuremoleküle vollständig chemisch synthetisiert werden. Man kann auch Genfragmente, z.B. aus Genen für Acetylcholinrezeptor-Untereinheiten aus Insekten, in das Gen von Interesse, z.B. ein Gen für eine Acetylcholinrezeptor-Untereinheit von Vertebraten, einführen. Dazu können Restriktionsschnittstellen ausgenutzt werden oder auch geeignete Restriktionsschnittstellen geschaffen werden, beispielsweise mit den Methoden der "site-directed mutagenesis" oder der PCR. Schließlich können Gene für Acetylcholinrezeptor-Untereinheiten von Interesse auch direkt mit den Methoden der "site-directed mutagenesis" oder der PCR verändert werden, um die gewünschten Eigenschaften und Strukturmerkmale zu erzielen. Auch die homologe Rekombination zwischen DNA-Sequenzen bietet eine Möglichkeit zur gezielten Veränderung der Gene.

Für PCR-Verfahren werden chemisch synthetisierte Oligonukleotide als Primer eingesetzt. Der Ausdruck "Oligonukleotid(e)", wie er hierin verwendet wird, bedeutet DNA-Moleküle, die aus 10 bis 50 Nukleotiden, vorzugsweise 15 bis 30 Nukleotiden, bestehen. Sie werden chemisch synthetisiert.

Mit Hilfe der erfindungsgemäßen Nukleinsäuren bzw. Acetylcholinrezeptor-Untereinheiten können neue Wirkstoffe für den Pflanzenschutz bzw. pharmazeutische Wirkstoffe für die Behandlung von Mensch und Tier, wie chemische Verbindungen, welche als Modulatoren, insbesondere als Agonisten oder Antagonisten, die Eigenschaften der erfindungsgemäßen Acetylcholinrezeptoren verändern, identifiziert werden. Dazu wird ein rekombinantes DNA-Molekül, das zumindest eine erfindungsgemäße Nukleinsäure umfasst, in eine geeignete Wirtszelle eingebracht. Die Wirtzelle wird in Gegenwart einer oder mehrerer Verbindungen unter Bedingungen kultiviert, die die Expression der erfindungsgemäßen Rezeptoren erlauben. Die Detektion veränderter Leitungseigenschaften ermöglicht das Auffinden beispielsweise insektizider Substanzen.

Veränderungen der Rezeptoreigenschaften wie z.B. Öffnung des Kanals, fehlende Öffnung des Kanals trotz Anwesenheit eines Agonisten in ausreichender Konzentration, veränderte Öffnungswahrscheinlichkeit oder -dauer führen zu entsprechenden Veränderungen des Ionenstroms durch den Kanal. Diese können z.B. mit elektrophysiologischen Methoden direkt verfolgt werden (Gopalakrishnan et al. 1995, Buisson et al. 1996, Stetzer et al. 1996, Ragozzino et al. 1997). Auch radioaktiv markierte Ionen wie z.B. ⁸⁶Rb-Ionen erlauben eine direkte Verfolgung des Ionenstroms (Gopalakrishnan et al. 1996). Es kann auch die aus dem Ionenstrom resultierende Veränderung des Membranpotentials mit spannungssensitiven Farbstoffen dargestellt werden. Biologische Spannungssensoren sind ebenfalls beschrieben. Veränderungen des Membranpotentials führen in Zellen weiterhin zu einer Vielzahl von physiologischen Veränderungen, die mittelbar oder unmittelbar nachweisbar sind, wie z.B. Öffnung, Schließung, veränderte Öffnungswahrscheinlichkeit oder -dauer von spannungsabhängigen Ionenkanälen. Diese können ebenfalls mit den oben beschriebenen Methoden detektiert werden. Falls der Ionenstrom durch den Acetylcholinrezeptor Calciumionen enthalten kann, oder falls der Ionenstrom durch einen sekundär geöffneten Kanal Calciumionen enthalten kann, kann die Konzentrationsänderung des freien intrazellulären Calciums z.B. mit Calcium-sensitiven Farbstoffen nachgewiesen werden (Stetzer et al. 1996, Delbono et al. 1997, Staudermann et al. 1998, Zhang et al. 1999). Weitere bekannte Verfahren zum Nachweis der Änderung der intrazellulären Calciumkonzentration sind die Verwendung biolumineszenter Proteine oder der Einsatz von Reportergen-Konstrukten.

Der Ausdruck "Agonist", wie er hierin verwendet wird, bezieht sich auf ein Molekül, das Acetylcholinrezeptoren aktiviert.

Der Ausdruck "Antagonist", wie er hierin verwendet wird, bezieht sich auf ein Molekül, nach dessen Bindung die Aktivierung des Rezeptors evtl. selbst nach Binden eines Agonisten ausbleibt.

Der Ausdruck "Modulator", wie er hierin verwendet wird, stellt den Oberbegriff zu Agonist bzw. Antagonist dar. Modulatoren können kleine organisch-chemische Moleküle, Peptide oder Antikörper sein, die an die erfindungsgemäßen Rezeptoren binden. Weiterhin können Modulatoren kleine organisch-chemische Moleküle, Peptide oder Antikörper sein, die an ein Molekül binden, welches wiederum an die erfindungsgemäßen Rezeptoren bindet, und dadurch deren biologische Aktivität beeinflusst. Modulatoren können Mimetika von natürlichen Substraten und Liganden darstellen.

Vorzugsweise handelt es sich bei den Modulatoren um kleine organisch-chemische Verbindungen.

### Erläuterungen zum Sequenzprotokoll und zu den Abbildungen:

SEQ ID NO: 1 zeigt einen Aminosäuresequenzbereich aus der α-Untereinheit von Torpedo californica;
SEQ ID NO: 2 zeigt die Nukleotidsequenz der α-Untereinheit gemäß Beispiel 1A);
SEQ ID NO: 3 zeigt die von SEQ ID NO:2 abgeleitete Aminosäuresequenz;
SEQ ID NO: 4 zeigt die Sequenz des Primers 1 aus Beispiel 1A);
SEQ ID NO: 5 zeigt die Sequenz des Primers 2 aus Beispiel 1A);
SEQ ID NO: 6 zeigt die Nukleotidsequenz der α-Untereinheit gemäß Beispiel 1B);
SEQ ID NO: 7 zeigt die von SEQ ID NO: 6 abgeleitete Aminosäuresequenz;
SEQ ID NO: 8 zeigt die Sequenz des Primers 1 aus Beispiel 1B);
SEQ ID NO: 9 zeigt die Sequenz des Primers 2 aus Beispiel 1B);
SEQ ID NO: 10 zeigt die Nukleotidsequenz der α-Untereinheit gemäß Beispiel 1C);
SEQ ID NO: 11 zeigt die von SEQ ID NO: 10 abgeleitete Aminosäuresequenz;
SEQ ID NO: 12 zeigt die Sequenz des Primers 1 aus Beispiel 1C);
SEQ ID NO: 13 zeigt die Sequenz des Primers 2 aus Beispiel 1C);
SEQ ID NO: 14 zeigt die Sequenz des Primers 1 für die Konstruktion des Vektors pBluescript KS⁺-delta SacI;
SEQ ID NO: 15 zeigt die Sequenz des Primers 2 für die Konstruktion des Vektors pBluescript KS⁺-delta SacI;
SEQ ID NO: 16 zeigt die Sequenz des Primers 3 aus Beispiel 1C);
SEQ ID NO: 17 zeigt die Sequenz des Primers 4 aus Beispiel 1C).

Die Abbildung 1A zeigt einen Sequenzvergleich von α-Untereinheiten nikotinischer Acetylcholinrezeptoren verschiedener Insekten- und Vertebratenspezies im Bereich der ligandenbindenden Domäne.

Die Sequenzen wurden mit Hilfe des ClustalX Programmes (Version 1.81) im Bereich der putativen ligandenbindenden Domäne (Changeux et al. 1992) aligniert. Die mit " _" versehenen Stellen markieren den Bereich, der zu SEQ ID NO: 1 homolog ist. " = " markiert den Bereich, der in SEQ ID NO: 6 ausgetauscht ist. Sternchen markieren den Bereich, der in SEQ ID NO: 10 ausgetauscht ist.

Die Abbildung 1B zeigt die Verwandtschaft der in der Abbildung 1A dargestellten Sequenzen gemäß dem Sequenzvergleichsprogramm njplotwin95 (Perrière er al. 1996) unter Standardeinstellungen. Das Programm gruppiert die Sequenzen, die am ähnlichsten sind. Der Abbildung kann entnommen werden, dass alle Sequenzen von Insekten-α-Untereinheiten untereinander verwandter sind als zu Sequenzen von α-Untereinheiten, die im Nervensystem von Hühnchen exprimiert werden, oder zu Sequenzen von α-Untereinheiten, die im Muskel von Torpedo oder im menschlichen Muskel exprimiert werden.

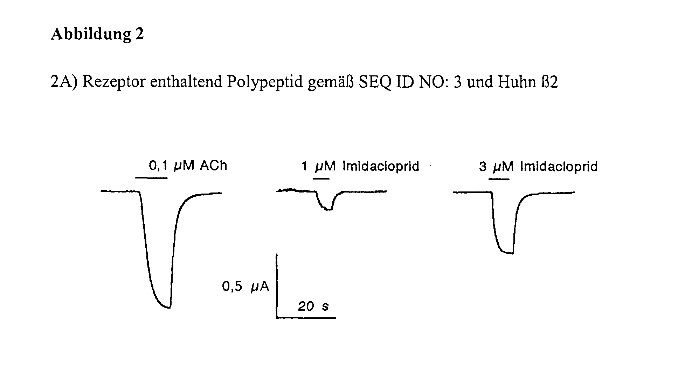

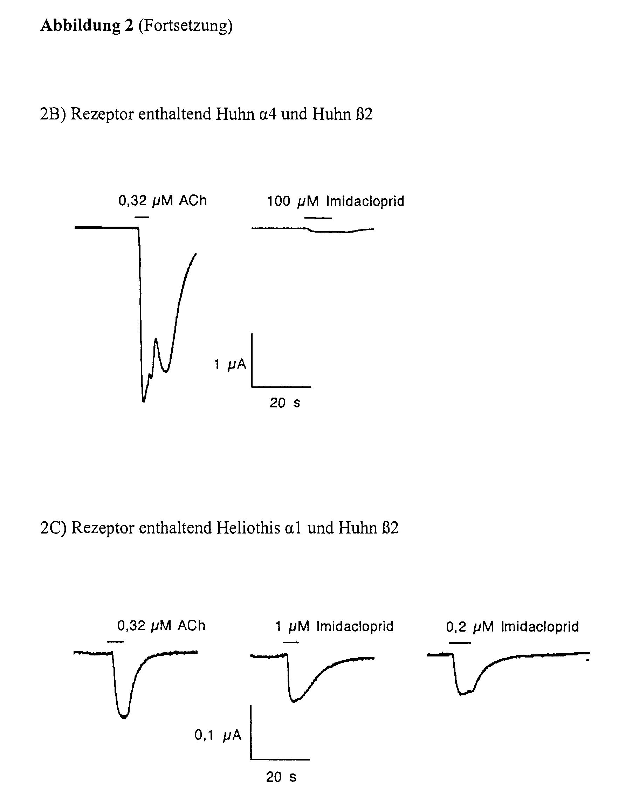

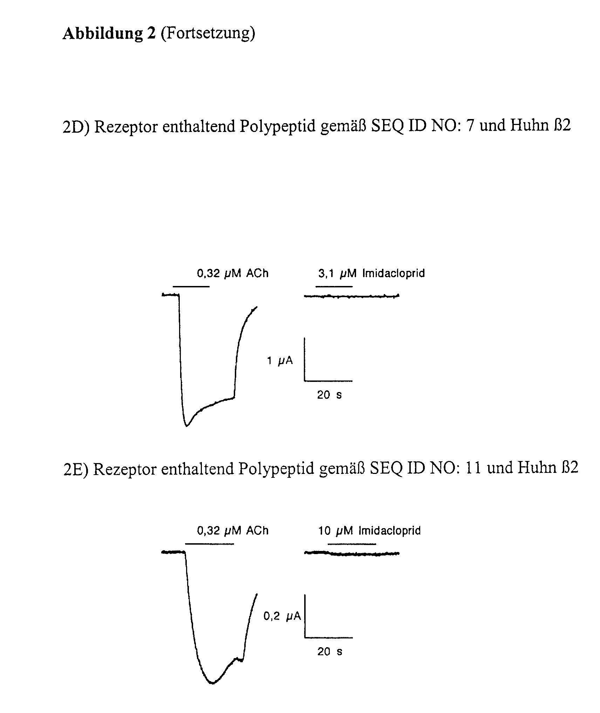

Die Abbildung 2 zeigt Strom-Zeit-Kurven, die von in Xenopus-Oocyten exprimierten Acetylcholinrezeptoren abgeleitet wurden. Die Experimente sind in Beispiel 2 beschrieben. Waagerechte Balken über den Kurven kennzeichnen die Zeiträume der Perfusion der Messkammer mit den über den Balken angegebenen Testlösungen. Der L-förmige Maßstab gibt die Zeit- (horizontal) und die Stromachse (vertikal) an.
A: Rezeptoren enthaltend Untereinheiten gemäß SEQ ID NO: 3 und Huhn β2
B: Rezeptoren enthaltend Huhn α4- und Huhn β2-Untereinheiten
C: Rezeptoren enthaltend Heliothis virescens α1- und Huhn β2-Untereinheiten
D: Rezeptoren enthaltend Untereinheiten gemäß SEQ ID NO: 7 und Huhn β2
E: Rezeptoren enthaltend Untereinheiten gemäß SEQ ID NO: 11 und Huhn β2

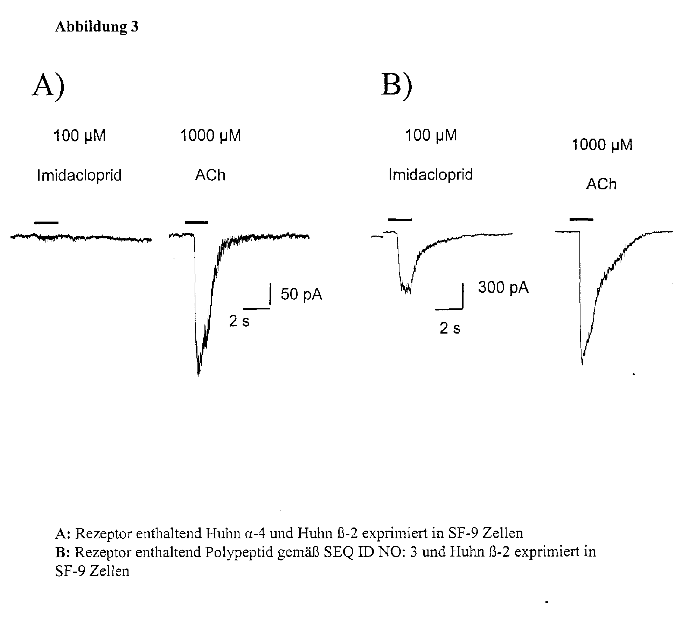

Die Abbildung 3 zeigt Strom-Zeit-Kurven, die von in Sf9 exprimierten Acetylcholinrezeptoren abgeleitet wurden. Die Experimente sind in Beispiel 3 beschrieben. Waagerechte Balken über den Kurven kennzeichnen die Zeiträume der Applikation der über den Balken angegebenen Testlösungen. Der Maßstab gibt die Zeit(horizontal) und die Stromachse (vertikal) an.
A: Rezeptoren enthaltend Huhn α4- und Huhn β2-Untereinheiten
B: Rezeptoren enthaltend Untereinheiten gemäß SEQ ID NO: 3 und Huhn β2

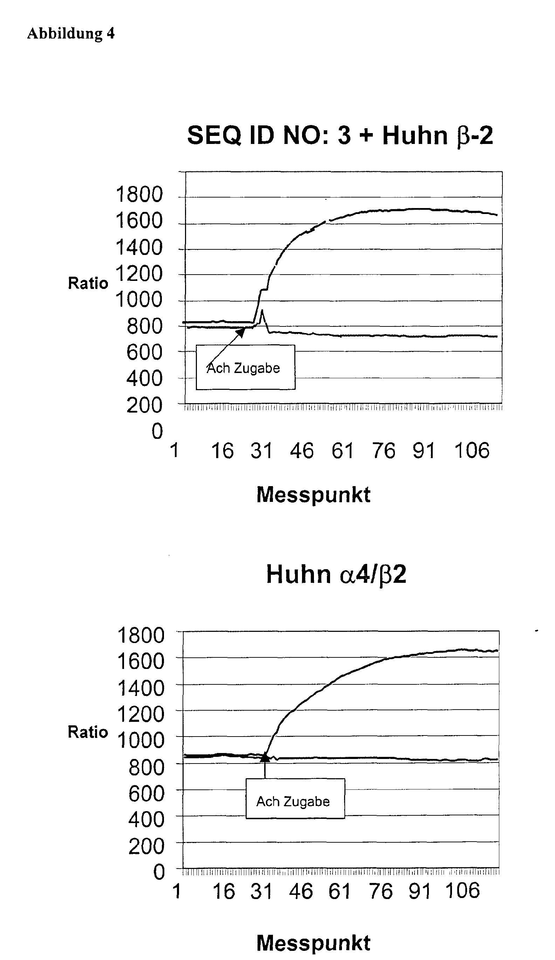

Die Abbildung 4 zeigt den Anstieg des intrazellulären Calciums in Sf9-Zellen, die die Rezeptoren gemäß SEQ ID NO: 3 und Huhn β2 (oben) bzw. Huhn α4- und Huhn β2-Untereinheiten (unten) exprimierten. Die Experimente sind in Beispiel 4 beschrieben. Die horizontale Skala gibt die Zeitachse wieder: Der Abstand zwischen zwei Messpunkten beträgt 250 ms. Die vertikale Achse gibt die relative Calcium-Konzentration in den Zellen auf einer nicht normierten Skala wieder. Die relative Calciumkonzentration wurde aus dem Verhältnis der Fluoreszenzaktivitäten der Zellen bei Bestrahlung mit Licht der Wellenlängen 340 nm und 380 nm gebildet. Reihe 1 (gekennzeichnet durch Liganden-induzierten Anstieg der Ca-Konzentration) kennzeichnet transfizierte Zellen, Reihe 2 nicht-transfizierte Kontroll-Zellen im gleichen Bildfeld.

### Beispiele:

### Allgemeines

Es wurden verschiedene Nukleinsäuren generiert, die für modifizierte α-Untereinheiten codieren.

Neben der Nukleinsäure gemäß SEQ ID NO: 2 wurden auch Nukleinsäuren auf Basis der Huhn α4-Untereinheit erzeugt, welche andere Bereiche aus dem ligandenbindenden Aminosäurebereich der Heliothis virescens α1-Untereinheit (SEQ ID NO: 6, 10) enthalten. Mit keiner dieser beiden anderen modifizierten α-Untereinheiten konnte die gestellte Aufgabe gelöst werden.

Bei den α-Untereinheiten gemäß SEQ ID NO: 7, 11 war beispielsweise keine Empfindlichkeit für Insektizide des Chlornikotinyl-Typs nachweisbar. Ihre pharmakologischen Eigenschaften entsprechen denen des Wildtyp Huhn α4/β2-Rezeptors und sind damit nicht für die oben genannte Fragestellung geeignet.

Um die guten Expressionseigenschaften der α4-Untereinheit aus dem Huhn mit der gewünschten insektentypischen Pharmakologie der Heliothis virescens α1-Untereinheit zu kombinieren, ist es von entscheidender Bedeutung, einen eng definierten Bereich innerhalb der Ligandenbindedomäne der α-Untereinheiten auszutauschen. Das Polypeptid gemäß SEQ ID NO: 3 enthält diesen Bereich.

### Beispiel 1

### Konstruktion der beschriebenen Nukleinsäuren

### Allgemeines

Die Manipulation von Polynukleotiden erfolgte nach Standardmethoden der rekombinanten DNA Technologie (Sambrook et al. 1989). Die bioinformatische Bearbeitung von Nukleotid- und Proteinsequenzen erfolgten mit dem Programmpaket GCG Version 10.0 (GCG Genetics Computer Group, Inc., Madison Wisconsin, USA).

### A) Konstruktion der Nukleinsäure gemäß SEQ ID NO: 2

a) Bei einem pBluescript KS⁺ (Stratagene, Heidelberg, Deutschland) wurde mittels Quickchange (Stratagene, Heidelberg, Deutschland) nach Angaben des Herstellers unter Verwendung der folgenden Oligonukleotide: SEQ ID NO: 14 (5'-GAACAAAAGCTGGAGGTCCACCGCGGTGGC-3') und SEQ ID NO: 15 (5'-GCCACCGCGGTGGACCTCCAGCTTTTGTTC-3') die SacI-Restriktionsschnittstelle entfernt.
b) Als Templat für eine Polymerase-Ketten-Reaktion (PCR) diente die cDNA der α1-Untereinheit des nikotinischen Acetylcholinrezeptors von Heliothis virescens (Genbank AJ000399) im Vektor pBluescript KS⁺ (10ng/µl). Als Primer wurden Oligonukleotide der Sequenz SEQ ID NO: 4 (5'-CACGTGCCCTCCGAGCTCATCTGGCGGCCGG-3') für das 5'-Ende des zu amplifizierenden Fragments und SEQ ID NO: 5 (5'-GTCATATGTCCACGAGCCGAAC-3') für das 3'-Ende des Fragments in einer Konzentration von jeweils 15 pmol/µl eingesetzt. Die verwendete Polymerase war PfuTurbo (Stratagene, Heidelberg, Deutschland). Betain wurde als 5 M Stammlösung in Wasser eingesetzt. Die Nukleotid-Stammlösung enthielt alle 4 Nukleotide in einer Konzentration von je 1mM.

| | | |
|---|---|---|
| Ansatz: | 51,4µl | H₂O |
| | 10 µl | 10x PfuTurbo Puffer (Stratagene, Heidelberg, Deutschland) |
| | 2 µl | Templat-DNA (20ng) |
| | 2 µl | d'NTP mix, jeweils 1mM |
| | 2 µl | Primer für das 5'-Ende |
| | 2 µl | Primer für das 3'-Ende |
| | 2,6 µl | Dimethylsulfoxid (wasserfrei) |
| | 26 µl | Betain |
| | 2 µl | PfuTurbo Polymerase (Stratagene, Heidelberg, Deutschland) |
| | | |
| PCR Prog.: | (1) 95°C | 1min |
| | | |
| | (2) 95°C | 30sec |
| | (3) 55°C | 30sec |
| | (4) 72°C | 30sec, 29 mal zurück zu (2) |
| | | |
| | (5) 4°C | Pause |

Nach der PCR wurde das Reaktionsprodukt mittels TOPO-TA Kit (Invitrogen, La Jolla, CA, USA) nach Angaben des Herstellers in einen TOPO-TA Vektor subkloniert. Eine Kolonie, die ein Plasmid mit dem amplifizierten Fragment enthielt, wurde mit Hilfe von Restriktionsverdaus identifiziert. Aus ihr wurde Plasmid-DNA mit üblichen Methoden gewonnen. Aus dieser DNA wurde ein SacI/NdeI-Fragment mit üblichen Methoden isoliert.

Parallel dazu wurde die cDNA der α4-Untereinheit des nikotinischen Acetylcholinrezeptors des Huhns (Genbank AJ250361) in den oben beschriebenen Vektor gemäß 1Aa (pBluescript KS⁺ ohne Sac I) über flankierende EcoRI-Schnittstellen kloniert. Dieses Plasmid wurde anschließend mit SacI und NdeI verdaut.

Das SacI/NdeI-Fragment wurde mit üblichen Methoden in die geöffnete cDNA der α4-Untereinheit des Huhns ligiert. Ein Aliquot des Ligationsansatzes wurde mit üblichen Methoden in kompetente E.coli Zellen des Stammes DH5α (Gibco, Karlsruhe, Deutschland) transformiert.

Eine Kolonie, die ein Plasmid mit dem einligierten Fragment enthielt, wurde mit Hilfe von Restriktionsverdaus identifiziert. Aus ihr wurde Plasmid-DNA mit üblichen Methoden gewonnen. Aus dieser DNA wurde ein BamHI/Eco47III-Fragment isoliert.

Parallel dazu wurde die cDNA der α4-Untereinheit des nikotinischen Acetylcholinrezeptors des Huhns mit BamHI und Eco47III verdaut. Die cDNA war kloniert in den Vektor pcDNA3.1⁺. Das BamHI/Eco47III-Fragment wurde mit üblichen Methoden in die geöffnete cDNA der α4-Untereinheit des Huhns ligiert. Ein Aliquot des Ligationsansatzes wurde in kompetente E.coli Zellen des Stammes DH5α transformiert.

Eine Kolonie, die ein Plasmid mit dem einligierten Fragment enthielt, wurde mit Hilfe von Restriktionsverdaus identifiziert. Aus ihr wurde Plasmid-DNA mit üblichen Methoden gewonnen. Diese Plasmid-DNA wurde für Injektionen in Xenopus-Oocyten verwendet.

### B) Konstruktion einer Nukleinsäure gemäß SEQ ID NO: 6

In die DNA der Huhn α4-Untereinheit (Genbank AJ250361) im Vektor gemäß 1Aa wurde durch Quickchange Mutagenese (Stratagene, Heidelberg, Deutschland) die sogenannte insektentypische Insertion aus der Heliothis virescens α1-Untereinheit (codierend für RHIDEARGTNVVELG) eingefügt. Die Mutagenese wurde nach Angaben des Herstellers mit Hilfe der folgenden Oligonukleotide durchgeführt:
SEQ ID NO: 8 (5'-GCTAAGATAGACTTGAGACACATCGATGAGGCTAGAGGAACCAACGTGG TAGAACTGGGTGTGGACCAACTGGACTACTGG-3') und SEQ ID NO: 13 (5'-CCAGTAGTCCAGTTGGTCCACACCCAGTTCTACCACGTTGGTTCCTCTAG CCTCATCGATGTGTCTCAAGTCTATCTTAGC-3')

### C) Konstruktion einer Nukleinsäure gemäß SEQ ID NO: 10

Die Nukleinsäure gemäß SEQ ID NO: 10 wurde ausgehend von der der Huhn α4-Untereinheit (Genbank AJ250361) im Vektor gemäß 1Aa durch eine zweistufige Quickchange Mutagenese (Stratagene, Heidelberg, Deutschland) nach Angaben des Herstellers erzeugt. Für die erste Reaktion fanden folgende Oligonukleotide Verwendung:
SEQ ID NO: 12 (5'-CAACAGCAAGAAATATGAATGCTGCGACGAGCCCTACCTTGATATAACTT TCAACTTCATTATCCGGAGGCTGCCGCTG-3') und SEQ ID NO: 17 (5'-CAGCGGCAGCCTCCGGATAATGAAGTTGAAAGTTATATCAAGGTAGGGC TCGTCGCAGCATTCATATTTCTTGCTGTTG-3'). Dieses Produkt wurde anschließend einer zweiten Quickchange Mutagenese mit folgenden Oligonukleotiden unterzogen: SEQ ID NO: 16 (5'-GC GGG GAG TGG GTC ATC TTAGAA GTC CCG GCC GTT CGC AAC GAA AAG TTT TAT ACA TGC TGC GAC GAG CCC TAC C-3') und SEQ ID NO: 17 (5'-G GTA GGG CTC GTC GCA GCA TGT ATA AAA CTT TTC GTT GCG AAC GGC CGG GAC TTC AATGAT GAC CCA CTC CCC GC-3')

Die Ergebnisse sind in der Abbildung 2 gezeigt. Rezeptoren, die nicht-modifizierte Huhn-α4-Untereinheiten und Huhn-β2-Untereinheiten umfassen, antworten auf die Zugabe von Acetylcholin, jedoch nicht auf die Zugabe von Imidacloprid mit ligandeninduzierter Leitfähigkeit (Abbildung 2B), während Rezeptoren, die nicht-modifizierte Heliothis-α1-Untereinheiten und Huhn-β2-Untereinheiten umfassen, sowohl für Acetylcholin als auch für Imidacloprid empfindlich sind (Abbildung 2C). Rezeptoren, die aus der Huhn-β2-Untereinheit und Polypeptiden gemäß SEQ ID NOs: 3, 7 bzw. 11 bestehen, sind alle funktionell, d.h. sie zeigen ligandeninduzierte Leitfähigkeit nach Zugabe von Acetylcholin (Abbildungen 2A, E, F). Schon dies ist im Hinblick auf die Ergebnisse von van den Beukel (van den Beukel 1998) überraschend. Nur die Rezeptoren, die aus der Huhn-β2-Untereinheit und Polypeptiden gemäß SEQ ID NO: 3 bestehen, sind jedoch empfindlich gegenüber Imidacloprid (Abbildung 2A).

### Beispiel 2

### Expression der modifizierten Acetylcholinrezeptoren in Xenopus-Oocyten

### Allgemeines

Um die Wirkung von Acetylcholin, Imidacloprid oder anderen potenziellen Agonisten der Acetylcholinrezeptoren auf die hergestellten modifizierten Rezeptoren zu charakterisieren, wurden elektrophysiologische Messungen an Xenopus-Oocyten durchgeführt. Die entsprechenden Methoden und Versuchsanordnungen sind in der Literatur an vielen Stellen beschrieben worden (s. z.B. Kettenmann & Grantyn, eds. 1992). Die Expression von klonierten oder rekombinant hergestellten Rezeptorgenen in Xenopus-Oocyten hat eine Reihe von technischen Vorteilen. Die Oocyten lassen sich durch einfache Injektion von mRNA oder cDNA zur Expression der entsprechenden Rezeptoren anregen und an diesen Zellen können die notwendigen elektrophysiologischen Messungen besonders einfach und bequem durchgeführt werden (z.B. Bertrand et al. 1992, Amar et al. 1993, Cooper et al. 1996).

### Expression der modifizierten Rezeptoren in Xenopus-Oocyten

Xenopus-Oocyten wurden isoliert und wie bereits beschrieben bereitgestellt (Bertrand et al. 1991). Am ersten Tag nach der Isolierung der Oocyten wurden jeweils 10 nl einer Lösung mit 2 ng eines entsprechenden cDNA-Expressionsvektors in die Zellkerne der Oocyten injiziert. Die Oocyten wurden 3 - 5 Tage bei 19°C in einem geeigneten Medium gehalten (BARTH-Lösung bestehend (in mM) aus NaCl 88, KCl 1, NaHCO₃ 2,4, MgSO₄ 0,82, Ca(NO₃)₂ 0,33, CaCl₂ 0,41, HEPES 10, pH 7,4). Nach dieser Zeit wurden die elektrophysiologischen Versuche durchgeführt.

### Elektrophysiologische Experimente

Elektrophysiologische Aufzeichnungen wurden unter Verwendung einer Dualelektroden-Spannungsklemme nach bewährten und bekannten Methoden durchgeführt (vgl. Bertrand et al. 1992). Jede Oocyte wurde einzeln in eine Messkammer platziert und mit zwei Mikroelektroden angestochen. Die Mikroelektroden sind fein ausgezogene Glaskapillaren, die mit einer geeigneten Salzlösung (z.B. 3M KCl oder 1,5M K-Acetat mit 100mM KCl) gefüllt wurden und dann einen Serienwiderstand von 0,3-1,2 M-Ohm aufweisen. Die Membranspannung wurde mit Hilfe des Voltage-Clamp-Verstärkers (TEC-00, Fa. npi, Tamm, Deutschland) auf -80mV festgesetzt und der Einwärtsstrom, der durch die Zellmembran fließt, wurde gemessen und mit einem Computer registriert. Die Messkammer wurde mit Frosch-Ringerlösung, enthaltend: 115 mM NaCl; 2,5 mM KCl; 1,8 mM CaCl₂; 10 mM HEPES; bei pH 7,4 (eingestellt mit NaOH) mit einer Fließgeschwindigkeit von 5 - 10 ml/Minute durchströmt. Um Acetylcholin, Imidacloprid oder eine andere Wirksubstanz an diesen Oocyten zu testen, wurde die Substanz in der vorgesehenen Konzentration der Frosch-Ringer-Lösung zugesetzt und die Perfusion der Messkammer wurde kurzfristig auf diese Testlösung umgestellt. Acetylcholin (Fluka, Buchs, Schweiz) wurde als Stammlösung bei -20°C aufbewahrt und unmittelbar vor dem Experiment der Messlösung zugegeben.

Alle modifizierten Rezeptoren, die auf Acetylcholin reagierten, wurden anschließend auch mit Imidacloprid getestet. Dabei ist am Auftreten eines zusätzlichen Einwärts-Stromsignals sogleich zu erkennen, ob die in der jeweiligen Oocyte ausgeprägte Rezeptorvariante von Imidacloprid aktiviert werden kann, oder nicht.

Um die Empfindlichkeit der modifizierten Rezeptoren auf Acetylcholin und Imidacloprid genauer zu charakterisieren, wurden Dosis-Wirkungs-Kurven aufgenommen, indem das oben beschriebene Experiment mit unterschiedlichen Konzentrationen der Substanz wiederholt wurde. Die Darstellung der relativen Signalstärken (bezogen auf die Strom-Antwort, die von einer Standarddosis Acetylcholin, hier meist 0,32 µM, ausgelöst wird) gegen die Konzentration der Testsubstanz erlaubt unmittelbar den Vergleich, welche Rezeptorvarianten gegenüber Imidacloprid besonders empfindlich sind indem erheblich geringere Konzentration an Imidacloprid erforderlich sind, um ein Stromsignal auszulösen.

### Beispiel 3

### Funktionelle Expression eines modifizierten Acetylcholinrezeptors in Sf9-Zellinien, enthaltend die modifizierte Untereinheit gemäß SEQ ID NO: 3

Spodoptera frugiperda 9 (Sf9) Zellen wurden gleichzeitig mit cDNA-Expressionsplasmiden, die für die modifizierte Heliothis/Huhn-Untereinheit und für die Huhn β2-Untereinheit codieren, unter Verwendung einer liposomalen Transfektionsreagenz (DAC-30, Eurogentec, Belgien) transfiziert. Als weitere cDNA wurde gleichzeitig ein Expressionskonstrukt für das grün fluoreszierende Protein aus Aequorea victoria transfiziert. Dies erlaubt eine einfache Identifizierung transfizierter Zellen, weil erfahrungsgemäß ein Großteil der Zellen, die eines der Konstrukte aufgenommen hat, auch die anderen aufgenommen hat. 24 bis 48 Stunden nach Transfektion wurden die Ströme durch die Zellmembran der Sf9-Zellen mit Ganz-Zell-Ableitungen gemessen. Dazu wurde die Potenzialdifferenz über die Zellmembran auf -70 mV konstant gehalten. Substanzapplikationen erfolgten mittels der U-Tube-reversed flow technique (Fenwick 1982). Das Volumen der Versuchskammer, die kontinuierlich mit Badlösung perfundiert wurde (3 ml/min), betrug weniger als 0,5 ml. Die Standardperfusionslösung (extrazelluläres Medium) hatte folgende Zusammensetzung (in mM): 150 NaCl, 4 KCl, 2 MgCl₂, 2 CaCl₂, 10 HEPES (pH 7,3). Die Pipettenlösung enthielt (in mM): 150 KCl, 10 HEPES 10 K-EGTA (pH 7,2). Die Mikroelektroden wurden am Elektrodenpuller (Zeitz, Deutschland) aus Borosilicatglasrohlingen (Außendurchmesser 1,6 mm, Hilgenberg, Deutschland) hergestellt. Der Widerstand der feuerpolierten Mikroelektroden lag bei Verwendung der obengenannten Pipetten- und Badlösung zwischen 4 und 6 MΩ. Alle Versuche wurden bei Raumtemperatur (22 - 25°C) mit einem L/M-EPC7 patchclamp-Verstärker (List electronic) durchgeführt. Die analogen Signale wurden mittels 8pole-Besselfilter auf 315 Hz gefiltert und mit 1 kHz digitalisiert. Zur Aufnahme und Analyse der Daten wurde die Software pClamp (Version 6.06) benutzt. Nach Erreichen des "giga-seals" wurden die schnellen Störkapazitäten (Pipettenkapazität) mit dem C-Fast-Kompensations-modus des EPC-7 kompensiert. Es wurde keine Kompensation des Serienwiderstands (Zellkapazität) vorgenommen.

Zur Überprüfung, ob die Expression der cDNAs, die für die modifizierte Heliothis/Huhn-Untereinheit gemäß SEQ ID NO: 3 und die Huhn β2-Untereinheit codieren, zur Produktion funktioneller Acetylcholinrezeptoren in den Zellen führte, wurde nach der oben beschriebenen Methode Ganz-Zell-Ableitungen durchgeführt und die Zellen dabei mit Acetylcholin (1000 µM) bzw. Imidacloprid (100 µM) stimuliert. Unmittelbar nach dem Stimulus konnten starke Einwärtsströme gemessen werden, die typisch für die Aktivierung von Ionenkanälen waren und zwar sowohl bei Applikation von 1000 µM Acetylcholin als auch bei Applikation von 100 µM Imidacloprid (Abb. 3). Um die Ergebnisse zu quantifizieren, wurde eine Versuchsreihe mit 5 Messungen durchgeführt. Dabei wurden die Einwärtsströme induziert durch 100 µM Imidacloprid verglichen mit den Einwärtsströmen ausgelöst durch 1000 µM Acetylcholin. Das Amplitudenverhältnis (maximale Amplitude der Strom/Zeit-Kurve bei Applikation von 100 µM Imidacloprid geteilt durch die maximale Amplitude der Strom/Zeit-Kurve bei Applikation von 1000 µM Acetylcholin) ist ein relatives Maß für die Empfindlichkeit eines Rezeptors für Imidacloprid. Dieses Amplitudenverhältnis betrug beim Rezeptor enthaltend Polypeptid gemäß SEQ ID NO: 3 und die Huhn-Untereinheit β2 0,46±0,09 (n=5 Zellen). Dagegen traten an Sf9-Zellen, die gleichzeitig mit cDNA-Expressionsplasmiden transfiziert wurden, die für die Huhn-Untereinheit α4 und die Huhn-Untereinheit β2 kodieren, nach Applikation von 100 µM Imidacloprid entweder keine oder nur sehr schwache Einwärtsströme auf. Das Amplitudenverhältnis dieser Rezeptoren betrug 0.05 + 0.06, (n= 5 Zellen). Nicht-transfizierte Kontroll-Zellen im gleichen Experiment, die durch das Fehlen der Fluoreszenz des grün fluoreszierenden Proteins identifiziert werden konnten, zeigten weder eine Antwort auf Acetylcholin noch auf Imidacloprid.

Diese Ergebnisse zeigen, dass die Acetylcholinrezeptor-α-Untereinheit mit einer Aminosäuresequenz gemäß SEQ ID NO: 3 zusammen mit der β2-Untereinheit aus dem Huhn einen funktionellen Rezeptor in Sf9-Zellen bildet, der sich pharmakologisch deutlich vom rekombinant exprimierten Huhn α4-β2-Rezeptor unterscheidet.

### Beispiel 4

### Nachweis der Aktivierung der zellulär exprimierten Acetylcholinrezeptoren enthaltend eine Untereinheit gemäß SEQ ID NO: 3 durch Agonisten mittels Calcium-Imaging

### Zellkultur und Gentransfer

Sf9-Zellen wurden in einer Mischung aus ¾ TC100 Medium (Gibco, Karlsruhe, Deutschland) + ¼ SF900 Medium (Gibco, Karlsruhe, Deutschland), 10 % foetalem Kälberserum, 0,1% Pluronic (Gibco, Karlsruhe, Deutschland) bei 27°C kultiviert. Für den Gentransfer wurde DAC-30 (Eurogentec) nach Angaben des Herstellers verwendet. Als weitere cDNA wurde gleichzeitig ein Expressionskonstrukt für das grün fluoreszierende Protein aus Aequorea victoria transfiziert. Dies erlaubt eine einfache Identifizierung transfizierter Zellen, weil erfahrungsgemäß ein Großteil der Zellen, die eines der Konstrukte aufgenommen hat, auch die anderen aufgenommen hat. 24 Stunden bis 48 Stunden nach dem Gentransfer wurden die Zellen in verschiedenen Dichten in Mikrotiterplatten ausgesät.

### Fura-2-Messungen

Die Veränderungen der intrazellulären Calcium-Konzentration wurden mit Fura-2 gemessen. Eine Stammlösung mit 2 mM Fura-2-acetoxymethylester (Sigma, München, Deutschland) in Dimethylsulfoxid (DMSO) wurde auf eine Endkonzentration von 10 µM in ¾ TC100 Medium (Gibco, Karlsruhe, Deutschland) + ¼ SF900 Medium (Gibco, Karlsruhe, Deutschland) mit 2% Rinderserumalbumin (Sigma, München, Deutschland) verdünnt. Die Zellen wurden in einer Mikrotiterplatte in dieser Lösung 45 bis 60 Minuten lang inkubiert. Anschließend wurden die Zellen zweimal in N-(2-Hydroxyethyl)piperazine-N'-(2-ethanesulfonic acid) (5 mM HEPES) gepufferter Calcium-Puffer (HEPES-gepufferte Salzlösung, pH 7,2 mit 84 mM CaCl₂) gewaschen. 100 µl Tyrodepuffer wurde in die Vertiefungen der Mikrotiterplatte gegeben und die Zellen wurden unter einem Fluoreszenzmikroskop (Axiovert, Zeiss, Jena, Deutschland) abwechselnd mit Licht der Wellenlänge 340 nm und 380 nm bestrahlt. Eine Serie von Videobildern (120 Bilder) mit einer Zeitauflösung von 250 msec wurde mit Hilfe eines TILL Imago CCD / Polychrom Bildanalyse Systems (T.I.L.L. Photonics, Martinsried, Deutschland) aufgenommen. Nach der Aufnahme von 30 Bildern wurden die Zellen durch Zugabe von 600 µl 2mM Acetylcholinchlorid in Calcium-Puffer stimuliert (Endkonzentration an Acetylcholin = 1mM, Pfeil in Abbildung 4). Anschließend wurden die Daten mit Hilfe der TILLVision Software (3.3, T.I.L.L. Photonics, Martinsried, Deutschland) analysiert: Die Zellen in einem Bildfeld wurden anhand der Expression des grün fluoreszierenden Proteins in eine transfizierte und eine nicht-transfizierte Population getrennt. Für jede Population getrennt wurde die Fluoreszenzintensität der Zellen bei Bestrahlung mit Licht der Wellenlänge 380 nm durch die entsprechende Intensität bei 340 nm geteilt und so ein Verhältnis gebildet, das den relativen Anstieg der Calcium-Konzentration auf einer nicht normierten Skala darstellt (ähnlich wie Grynkiewicz et al. 1985).

Die Ergebnisse (Abb. 4) zeigen, dass sich Acetylcholinrezeptoren enthaltend eine Untereinheit gemäß SEQ ID NO: 3 in Zellkulturzellen funktional exprimieren lassen und ihre Stimulation zu einem Anstieg der Calcium-Konzentration in der Zelle führt.

### Literaturverzeichnis

Amar et al. 1995, A nicotinic acetylcholine receptor subunit from insect brain forms a non-desensitizing homo-oligomeric nicotinic acetylcholine receptor when expressed in Xenopus oocytes, Neuroscience Letters 199, 107-110.

Amar et al. 1993, Agonist pharmacology of the neuronal a7 nicotinic receptor expressed in Xenopus oocytes, FEBS Lett. 327, 284-288.

Bertrand et al. 1994, Physiological properties of neuronal nicotinic receptors reconstituted from the vertebrate beta 2 subunit and Drosophila alpha subunits, Eur. J. Neurosci. 6, 869-75.

Bertrand et al. 1992, Pharmacological properties of the homomeric alpha-7 receptor, Neurosci. Lett. 146, 87-90.

Bertrand et al. 1991, Methods in Neuroscience 4 , New York, Academic Press, 174-193.

Breer et al. 1987, Molecular properties and functions of insect acetylcholine receptors, J. Insect Physiol. 33, 771-790.

Buckingham et al. 1997, Imidacloprid actions on insect neuronal acetylcholine receptors, J. Exp. Biol. 200, 2685-2692.

Buisson et al. 1996, Human α3/β4 Neuronal Nicotinic Acetylcholine Receptor in HEK-293 cells: A Patch-Clamp Study, J Neuroscience 16, 7880-7891.

Changeux et al. 1992, The functional architecture of the nicotinic acetylcholine receptor explored by affinity labelling and site-directed mutagenesis, Quarterly Review of Biophysics 25, 395-432.

Claudio et al. 1983, Nucleotide and deduced amino acid sequences of Torpedo califomica acetylcholine receptor γ subunit, Proc. Natl. Acad. Sci. USA 80, 1111-1115.

Cooper et al. 1996, Pharmacology of the nicotinic acetylcholine receptor from fetal rat muscle expressed in Xenopus oocytes, Eur. J. Pharmacol. 309, 287-298.

Delbono et al. 1997, Activation of the Recombinant Human α7 Nicotinic Acetylcholine Receptor Significantly Raises Intracellular Free Calcium; J. Pharmacol. Exp. Therapeut. 280, 428-438.

Devillers-Thiery et al. 1983, Complete mRNA coding sequence of the acetylcholine binding α-subunit of Torpedo marmorata acetylcholine receptor: a model for the transmembrane organization of the polypeptide chain, Proc. Natl. Acad. Sci. USA 80, 2067-2071.

Eastham et al. 1998, Characterisation of a nicotinic acetylcholine receptor from the insect Manduca sexta, Eur. J. Neurosci 10, 879-889.

Elgoyhen et al. 1997, US Pat. No. 5,683,912.

Gopalakrishnan et al. 1995, Stable Expression and Pharmacological Properties of the Human α7 nicotinic acetylcholine receptor, Eur. J. Pharmacol. 290, 237-246.

Gopalakrishnan et al. 1996, Stable Expression and Pharmacological Properties of the Human Neuronal Nicotinic Acetylcholine α4/β2 receptor, J. Pharmacol. Exp. Therapeut. 276, 289-297.

Grynkiewicz et al. 1985, A new generation of Ca2+ indicators with greatly improved fluorescence properties, J. Biol. Chem. 260, 3440-3450.

Heinemann et al. 1997, US Pat. No 5,591,590.

Hermans-Borgmeyer et al. 1986, Primary structure of a developmentally regulated nicotinic acetylcholine receptor protein from Drosophila, EMBO J. 5, 1503-1508.

Hermsen et al. 1998, Neuronal nicotinic receptors in the locust Locusta migratoria. Cloning and expression, J. Biol. Chem. 17, 18394-404.

Huang et al. 1999, Molecular characterization and imidacloprid selectivity of nicotinic acetylcholine receptor subunits from the peach-potato aphid myzus persicae J. Neurochem. 73, 380-389.

Jespersen et al. 1997, Efficient Non-PCR-Mediated Overlap Extension of PCR Fragments by Exonuclease "End Polishing", Biotechniques, 23, 48.

Lansdell et al. 1997, Temperature-sensitive expression of Drosophila neuronal nicotinic acetylcholine receptors, J. Neurochem. 68, 1812-9.

Lansdell et al. 2000, The influence of nicotinic receptor subunit composition upon agonist, alpha-bungarotoxin and insecticide (imidacloprid) binding affinity, Neuropharmacology 39,671-9.

Lindstrom et al. 1997, US Pat. No. 5,599,709.

Marshall et al. 1990, Sequence and functional expression of a single α subunit of an insect nicotinic acetylcholine receptor, EMBO J. 9, 4391-4398.

Matsuda et al. 1998, Effects of the α subunit on imidacloprid sensitivity of recombinant nicotinic acetylcholine receptors, Br. J. Pharmacol. 123, 518-524.

Noda et al. 1982, Primary structure of α-subunit precursor of Torpedo californica acetylcholine receptor deduced from cDNA sequence, Nature 299, 793-797.

Noda et al. 1983a, Primary structures of β- and δ-subunit precursor of Torpedo califomica acetylcholine receptor deduced from cDNA sequences, Nature 301, 251-255.

Noda et al. 1983b, Structural homology of Torpedo californica acetylcholine receptor subunits, Nature 302, 528-532.

Ortells et al. 1995, Evolutionary history of the ligand-gated ion-channel superfamily of receptors, Trends in Neuroscience 18, 121-127.

Perrière et al. 1996, WWW-Query: An on-line retrieval system for biological sequence banks, Biochimie 78, 364-369

Ragozzino et al. 1997, Functional Properties of Neuronal Nicotinic Acetylcholine Receptor Channels Expressed in Transfected Human Cells, Eur. J. Neurosci. 9, 480-488.

Sawruk et al. 1990a, Heterogeneity of Drosophila nicotinic acetylcholine receptors: SAD, a novel developmentally regulated α-subunit. EMBO J. 9, 2671-2677.

Sawruk et al. 1990b, SBD, a novel structural subunit of the Drosophila nicotinic acetylcholine receptor, shares its genomic localization with two α-subunits, FEBS Lett. 273, 177-181.

Schloß et al. 1988, Neuronal acetylcholine receptors of Drosophila: the ARD protein is a component of a high-affinity α-bungarotoxin binding complex, EMBO J 7, 2889-2984.

Schoepfer et al. 1990, Brain alpha-bungarotoxin binding protein cDNAs and MAbs reveal subtypes of this branch of the ligand-gated ion channel gene superfamily; Neuron 5, 35-48.

Schulz et al. 1998, Dα3, a new functional α-subunit of nicotinic acetylcholine receptors from Drosophila, J. Neurochem. 71, 853-862.

Schulz et al. 2000, Neuronal nicotinic acetylcholine receptors from Drosophila: two different types of alpha subunits coassemble within the same receptor complex, J. Neurochem. 74, 2537-46.

Sgard et al. 1998, Cloning and Functional Characterizsation of Two Novel Nicotinic Acetylcholine Receptor α subunits form the Insect Pest Myzus persicae; J. Neurochem 71, 903-912.

Staudermann et al. 1998, Characterizsation of Human Recombinant Nicotinic Acetylcholine Receptor Subunit α2β4, α3β4 and α4β4Combinations Stably Expressed in HEK-293 Cells, J. Pharmacol. Exp. Therapeut. 284, 777-789.

Stetzer et al. 1996, Stable expression in HEK-293 cells of the rat a3/b4 subtype of neuronal nicotinic acetylcholine receptor, FEBS Lett. 397, 39-44.

Thompson et al. 1997, The ClustalX windows interface: flexible strategies for multiple sequence alignment aided by quality analysis tools, Nucleic Acids Research 24, 4876-4882.

Van den Beukel 1998, Species- and subtype-specific interactions of cholinesterase inhibitors with acetylcholine receptors, Dissertation Utrecht University, ISBN 90-393-1737-2.

Zhang et al. 1999, Activation and Ca2+ Permeation of Stably Transfected α3/β4 Neuronal Nicotinic Acetylcholine receptor; Mol. Pharmacol. 55, 970-981.

## Patentansprüche

1. Modifizierte Acetylcholinrezeptor-Untereinheit, **dadurch gekennzeichnet, dass** zumindest eine Aminosäure im Bereich einer α-Untereinheit eines Vertebraten-Acetylcholinrezeptors, welcher zu der Aminosäuresequenz gemäß SEQ ID NO: 1 homolog ist, durch eine Aminosäure, die an der identischen Position in dem entsprechenden Bereich einer α-Untereinheit eines Insekten-Acetylcholinrezeptors vorkommt, ersetzt ist.

2. Acetylcholinrezeptor-Untereinheit gemäß Anspruch 1, **dadurch gekennzeichnet, dass** zumindest vier Aminosäuren der α -Untereinheit eines Vertebraten-Acetylcholinrezeptors durch die entsprechende Anzahl von Aminosäuren einer α-Untereinheit eines Insekten-Acetylcholinrezeptors ersetzt sind.

3. Acetylcholinrezeptor-Untereinheit gemäß Anspruch 1, **dadurch gekennzeichnet, dass** zumindest sieben Aminosäuren der α-Untereinheit eines Vertebraten-Acetylcholinrezeptors durch die entsprechende Anzahl von Aminosäuren einer α-Untereinheit eines Insekten-Acetylcholinrezeptors ersetzt sind.

4. Acetylcholinrezeptor-Untereinheit gemäß Anspruch 1, **dadurch gekennzeichnet, dass** der gesamte besagte Bereich der α -Untereinheit eines Vertebraten-Acetylcholinrezeptors durch den entsprechenden Bereich einer α-Untereinheit eines Insekten-Acetylcholinrezeptors ersetzt ist.

5. Acetylcholinrezeptor-Untereinheit gemäß einem der Ansprüche 1 bis 4, **dadurch gekennzeichnet, dass** es sich bei der α-Untereinheit eines Vertebraten-Acetylcholinrezeptors um neuronale Untereinheiten von Maus, Ratte, Huhn, Zebrafisch, Rhesusaffe, Rind oder Schwein handelt.

6. Acetylcholinrezeptor-Untereinheit gemäß einem der Ansprüche 1 bis 5, **dadurch gekennzeichnet, dass** es sich bei der α-Untereinheit eines Insekten-Acetylcholinrezeptors um die α2-Untereinheit oder die α3-Untereinheit von Myzus persicae, oder um die α1-Untereinheit von Heliothis virescens oder Manduca sexta, oder um die α1-, α2- oder α3-Untereinheit von Drosophila melanogaster handelt.

7. Acetylcholinrezeptor-Untereinheit gemäß Anspruch 1, **dadurch gekennzeichnet, dass** sie die Aminosäuresequenz gemäß SEQ ID NO: 3 umfasst.

8. Acetylcholinrezeptor umfassend eine Acetylcholinrezeptor-Untereinheit gemäß einem der Ansprüche 1 bis 7.

9. Acetylcholinrezeptor gemäß Anspruch 8, **dadurch gekennzeichnet, dass** er zusätzlich eine β-Untereinheit von Maus, Ratte, Huhn, Zebrafisch, Rhesusaffe, Rind oder Schwein umfasst.

10. Nukleinsäure umfassend eine Nukleotidsequenz, die für eine Acetylcholinrezeptor-Untereinheit gemäß einem der Ansprüche 1 bis 7 codiert.

11. Nukleinsäure gemäß Anspruch 10, **dadurch gekennzeichnet, dass** es sich um einzelsträngige oder doppelsträngige DNA oder RNA handelt.

12. Nukleinsäure gemäß Anspruch 11, **dadurch gekennzeichnet, dass** es sich um Fragmente genomischer DNA oder cDNA handelt.

13. Nukleinsäure gemäß Anspruch 10, **dadurch gekennzeichnet, dass** die Nukleotidsequenz der Sequenz gemäß SEQ ID NO: 2 entspricht.

14. DNA-Konstrukt umfassend eine Nukleinsäure gemäß einem der Ansprüche 10 bis 13 und einen heterologen Promotor.

15. Vektor umfassend eine Nukleinsäure gemäß einem der Ansprüche 10 bis 13 oder ein DNA-Konstrukt gemäß Anspruch 14.

16. Vektor gemäß nach Anspruch 15, **dadurch gekennzeichnet, dass** die Nukleinsäure funktionell mit regulatorischen Sequenzen verknüpft ist, die die Expression der Nukleinsäure in pro- oder eukaryotischen Zellen gewährleisten.

17. Wirtszelle enthaltend eine Nukleinsäure gemäß einem der Ansprüche 10 bis 13, ein DNA-Konstrukt gemäß Anspruch 14 oder einen Vektor gemäß Anspruch 15 oder 16.

18. Wirtszelle gemäß Anspruch 17, **dadurch gekennzeichnet, dass** es sich um eine prokaryotische Zelle, insbesondere um E. coli, handelt.

19. Wirtszelle gemäß Anspruch 17, **dadurch gekennzeichnet, dass** es sich um eine eukaryotische Zelle, insbesondere um eine Säuger- oder Insektenzelle, handelt.

20. Verfahren zum Herstellen einer Acetylcholinrezeptor-Untereinheit gemäß einem der Ansprüche 1 bis 7, umfassend
a) das Kultivieren einer Wirtszelle gemäß einem der Ansprüche 17 bis 19 unter Bedingungen, die die Expression der Nukleinsäure gemäß einem der Ansprüche 10 bis 13 gewährleisten, oder
b) das Exprimieren einer Nukleinsäure gemäß einem der Ansprüche 10 bis 13 in einem in vitro-System, und
c) die Gewinnung des Polypeptids aus der Zelle, dem Kulturmedium oder dem in vitro-System.

21. Verfahren zum Herstellen einer Nukleinsäure gemäß einem der Ansprüche 10 bis 13, umfassend die folgenden Schritte:
(a) Vollständige chemische Synthese auf an sich bekannte Weise, oder
(b) chemische Synthese von Oligonukleotiden und Amplifizierung der besagten Nukleinsäure mittels PCR.

22. Verfahren zum Auffinden von Wirkstoffen für den Pflanzenschutz oder von pharmazeutischen Wirkstoffen für die Behandlung von Mensch oder Tier umfassend die folgenden Schritte:
(a) Bereitstellen einer Wirtszelle gemäß einem der Ansprüche 17 bis 19,
(b) Kultivieren der Wirtszelle in der Gegenwart einer oder mehrerer chemischen Verbindungen, und
(c) Detektieren veränderter Leitungseigenschaften der Acetylcholinrezeptoren.

23. Verwendung einer Acetylcholinrezeptor-Untereinheit gemäß einem der Ansprüche 1 bis 7, eines Acetylcholinrezeptors gemäß Anspruch 8 oder 9, einer Nukleinsäure gemäß einem der Ansprüche 10 bis 13, eines DNA-Konstrukts gemäß Anspruch 14, eines Vektors gemäß Anspruch 15 oder 16, oder einer Wirtszelle gemäß einem der Ansprüche 17 bis 19 zum Auffinden neuer Wirkstoffe für den Pflanzenschutz oder von pharmazeutischen Wirkstoffen für die Behandlung von Mensch oder Tier.


SEQUENZPROTOKOLL

<110> Bayer Aktiengesellschaft

<120> Acetylcholinrezeptor-Untereinheiten

<130> Le A 34 821

<140>
<141>

<150> DE 100 42 177.6
<151> 2000-08-28

<160> 17

<170> PatentIn Ver. 2.1

<210> 1
<211> 45
<212> PRT
<213> Torpedo californica

<400> 1
Asp Phe Ala Ile Val His Met Thr Lys Leu Leu Leu Asp Tyr Thr Gly
  1               5                  10                  15

Lys Ile Met Trp Thr Pro Pro Ala Ile Phe Lys Ser Tyr Cys Glu Ile
             20                  25                  30

Ile Val Thr His Phe Pro Phe Asp Gln Gln Asn Cys Thr
         35                  40                  45

<210> 2
<211> 1869
<212> DNA
<213> Künstliche Sequenz

<220>
<221> CDS
<222> (1)..(1866)

<220>
<223> Beschreibung der künstlichen Sequenz: Modifizierte
      alpha 4-Untereinheit des nikotinischen
      Acetylcholinrezeptors des Huhns

<400> 2
atg gga ttt ctc gtg tcg aag gga aac ctc ctc ctc ctg ctg tgt gcc     48
Met Gly Phe Leu Val Ser Lys Gly Asn Leu Leu Leu Leu Leu Cys Ala
1               5                   10                  15

agc atc ttc ccc gct ttc ggc cac gtg gaa acg cga gcc cat gcg gag     96
Ser Ile Phe Pro Ala Phe Gly His Val Glu Thr Arg Ala His Ala Glu
            20                  25                  30

gag cgc ctc ctg aag aaa ctc ttc tcc ggg tat aac aag tgg tcc cgt     144
Glu Arg Leu Leu Lys Lys Leu Phe Ser Gly Tyr Asn Lys Trp Ser Arg
        35                  40                  45

ccc gtc gcc aac att tcg gat gtg gtc ctg gtc cgc ttc ggc ttg tcc     192
Pro Val Ala Asn Ile Ser Asp Val Val Leu Val Arg Phe Gly Leu Ser
    50                  55                  60

ata gcc cag ctc atc gat gtt gat gag aag aac caa atg atg acc aca     240
Ile Ala Gln Leu Ile Asp Val Asp Glu Lys Asn Gln Met Met Thr Thr
65                  70                  75                  80

aat gtg tgg gtg aag cag gag tgg cac gac tac aag ctg cgc tgg gac     288
Asn Val Trp Val Lys Gln Glu Trp His Asp Tyr Lys Leu Arg Trp Asp
                85                  90                  95

ccc cag gag tat gaa aac gtc aca tcc atc cga atc ccc tca gag ctc     336
Pro Gln Glu Tyr Glu Asn Val Thr Ser Ile Arg Ile Pro Ser Glu Leu
            100                 105                 110

atc tgg cgg ccg gac ata gtc ctc tac aac aat gcc gac ggc aac ttc     384
Ile Trp Arg Pro Asp Ile Val Leu Tyr Asn Asn Ala Asp Gly Asn Phe
        115                 120                 125

gag gta acg ctg gcg acg aag gcg act ttg aat tat acg gga cgt gtg     432
Glu Val Thr Leu Ala Thr Lys Ala Thr Leu Asn Tyr Thr Gly Arg Val
    130                 135                 140

gag tgg cgc ccg ccg gct atc tac aag tcc tcg tgc gag atc gac gtg     480
Glu Trp Arg Pro Pro Ala Ile Tyr Lys Ser Ser Cys Glu Ile Asp Val
145                 150                 155                 160

gaa tac ttc ccg ttc gac cag cag acg tgc gtc atg aag ttc ggc tcg     528
Glu Tyr Phe Pro Phe Asp Gln Gln Thr Cys Val Met Lys Phe Gly Ser
                165                 170                 175

tgg aca tat gac aaa gct aag ata gac ttg gtg agc atg cat agc cat     576
Trp Thr Tyr Asp Lys Ala Lys Ile Asp Leu Val Ser Met His Ser His

180                 185                 190

gtg gac caa ctg gac tac tgg gaa agc ggg gag tgg gtc atc att aat     624
Val Asp Gln Leu Asp Tyr Trp Glu Ser Gly Glu Trp Val Ile Ile Asn
        195                 200                 205

gcc gtg ggc aat tac aac agc aag aaa tat gaa tgc tgc aca gag atc     672
Ala Val Gly Asn Tyr Asn Ser Lys Lys Tyr Glu Cys Cys Thr Glu Ile
    210                 215                 220

tac cct gat ata act tac tcc ttc att atc cgg agg ctg ccg ctg ttc     720
Tyr Pro Asp Ile Thr Tyr Ser Phe Ile Ile Arg Arg Leu Pro Leu Phe
225                 230                 235                 240

tac aca atc aat ttg atc att ccc tgc ctg ctt atc tcc tgc ttg act     768
Tyr Thr Ile Asn Leu Ile Ile Pro Cys Leu Leu Ile Ser Cys Leu Thr
                245                 250                 255

gtc ctg gtc ttc tac cta ccc tct gag tgc gga gag aag ata acc ttg     816
Val Leu Val Phe Tyr Leu Pro Ser Glu Cys Gly Glu Lys Ile Thr Leu
            260                 265                 270

tgc atc tct gtg ctg cta tcc ctc acg gtg ttc ctg ctg ctc atc aca     864
Cys Ile Ser Val Leu Leu Ser Leu Thr Val Phe Leu Leu Leu Ile Thr
        275                 280                 285

gag atc atc cct tct acc tcc ctg gtc atc ccc ctg ata gga gag tat     912
Glu Ile Ile Pro Ser Thr Ser Leu Val Ile Pro Leu Ile Gly Glu Tyr
    290                 295                 300

ctg ctc ttc acc atg ata ttt gtc acc ttg tct atc atc atc act gtc     960
Leu Leu Phe Thr Met Ile Phe Val Thr Leu Ser Ile Ile Ile Thr Val
305                 310                 315                 320

ttt gtg ctc aac gta cac cac cgt tca cca cgt acc cac acg atg cct     1008
Phe Val Leu Asn Val His His Arg Ser Pro Arg Thr His Thr Met Pro
                325                 330                 335

gac tgg gtg agg agg gtc ttc ctt gac ata gtc cca cgt ctc ctc ttc     1056
Asp Trp Val Arg Arg Val Phe Leu Asp Ile Val Pro Arg Leu Leu Phe
            340                 345                 350

atg aag cgg ccc tcc aca gtg aaa gac aat tgc aag aag ctt att gaa     1104
Met Lys Arg Pro Ser Thr Val Lys Asp Asn Cys Lys Lys Leu Ile Glu
        355                 360                 365

tct atg cac aaa cta acc aac tca cca agg ctt tgg tct gag acc gac     1152
Ser Met His Lys Leu Thr Asn Ser Pro Arg Leu Trp Ser Glu Thr Asp

370                 375                 380

atg gag ccc aac ttc act acc tca tcc tcc ccc agc ccc cag agt aat     1200
Met Glu Pro Asn Phe Thr Thr Ser Ser Ser Pro Ser Pro Gln Ser Asn
385                 390                 395                 400

gaa cct tca ccc aca tct tcc ttc tgt gcc cac ctt gag gag cca gcc     1248
Glu Pro Ser Pro Thr Ser Ser Phe Cys Ala His Leu Glu Glu Pro Ala
                405                 410                 415

aaa cct atg tgc aaa tcc cct tct gga cag tac tca atg ctg cac cct     1296
Lys Pro Met Cys Lys Ser Pro Ser Gly Gln Tyr Ser Met Leu His Pro
            420                 425                 430

gag ccc cca cag gtg acg tgt tcc tct ccg aag ccc tcc tgc cac ccc     1344
Glu Pro Pro Gln Val Thr Cys Ser Ser Pro Lys Pro Ser Cys His Pro
        435                 440                 445

ctg agt gac acc cag acc aca tct atc tca aaa ggc aga tcg ctc agt     1392
Leu Ser Asp Thr Gln Thr Thr Ser Ile Ser Lys Gly Arg Ser Leu Ser
    450                 455                 460

gtt cag cag atg tac agc ccc aat aag aca gag gaa ggg agc atc cgc     1440
Val Gln Gln Met Tyr Ser Pro Asn Lys Thr Glu Glu Gly Ser Ile Arg
465                 470                 475                 480

tgt agg tcc cga agc atc cag tac tgt tac ctg cag gag gac tct tcc     1488
Cys Arg Ser Arg Ser Ile Gln Tyr Cys Tyr Leu Gln Glu Asp Ser Ser
                485                 490                 495

cag acc aat ggc cac tct agt gcc tct cca gcg tcg cag cgc tgc cac     1536
Gln Thr Asn Gly His Ser Ser Ala Ser Pro Ala Ser Gln Arg Cys His
            500                 505                 510

ctc aat gaa gag cag ccc cag cac aag ccc cac cag tgc aag tgt aag     1584
Leu Asn Glu Glu Gln Pro Gln His Lys Pro His Gln Cys Lys Cys Lys
        515                 520                 525

tgc aga aag gga gag gca gct ggc aca ccg act caa gga agc aag agc     1632
Cys Arg Lys Gly Glu Ala Ala Gly Thr Pro Thr Gln Gly Ser Lys Ser
    530                 535                 540

cac agc aac aaa gga gaa cac ctc gtg ctg atg tcc cca gcc ctg aag     1680
His Ser Asn Lys Gly Glu His Leu Val Leu Met Ser Pro Ala Leu Lys
545                 550                 555                 560

ctg gcg gtg gaa ggg gtc cac tac att gca gac cac ctg cga gca gaa     1728
Leu Ala Val Glu Gly Val His Tyr Ile Ala Asp His Leu Arg Ala Glu

565                 570                 575

gat gca gat ttc tca gtg aag gaa gac tgg aag tac gta gca atg gtc    1776
Asp Ala Asp Phe Ser Val Lys Glu Asp Trp Lys Tyr Val Ala Met Val
            580                 585                 590

att gac cgg atc ttt ctc tgg atg ttc atc atc gtg tgt ttg ctg ggg    1824
Ile Asp Arg Ile Phe Leu Trp Met Phe Ile Ile Val Cys Leu Leu Gly
        595                 600                 605

acc gtt ggg ctc ttc ctc ccg ccg tgg ctg gca gga atg atc taa        1869
Thr Val Gly Leu Phe Leu Pro Pro Trp Leu Ala Gly Met Ile
    610                 615                 620

<210> 3
<211> 622
<212> PRT
<213> Künstliche Sequenz
<223> Beschreibung der künstlichen Sequenz: Modifizierte
      alpha 4-Untereinheit des nikotinischen
      Acetylcholinrezeptors des Huhns

<400> 3
Met Gly Phe Leu Val Ser Lys Gly Asn Leu Leu Leu Leu Leu Cys Ala
  1               5                  10                  15

Ser Ile Phe Pro Ala Phe Gly His Val Glu Thr Arg Ala His Ala Glu
             20                  25                  30

Glu Arg Leu Leu Lys Lys Leu Phe Ser Gly Tyr Asn Lys Trp Ser Arg
         35                  40                  45

Pro Val Ala Asn Ile Ser Asp Val Val Leu Val Arg Phe Gly Leu Ser
     50                  55                  60

Ile Ala Gln Leu Ile Asp Val Asp Glu Lys Asn Gln Met Met Thr Thr
 65                  70                  75                  80

Asn Val Trp Val Lys Gln Glu Trp His Asp Tyr Lys Leu Arg Trp Asp
                 85                  90                  95

Pro Gln Glu Tyr Glu Asn Val Thr Ser Ile Arg Ile Pro Ser Glu Leu
            100                 105                 110

Ile Trp Arg Pro Asp Ile Val Leu Tyr Asn Asn Ala Asp Gly Asn Phe
        115                 120                 125

Glu Val Thr Leu Ala Thr Lys Ala Thr Leu Asn Tyr Thr Gly Arg Val
    130                 135                 140

Glu Trp Arg Pro Pro Ala Ile Tyr Lys Ser Ser Cys Glu Ile Asp Val
145                 150                 155                 160

Glu Tyr Phe Pro Phe Asp Gln Gln Thr Cys Val Met Lys Phe Gly Ser
                165                 170                 175

Trp Thr Tyr Asp Lys Ala Lys Ile Asp Leu Val Ser Met His Ser His
            180                 185                 190

Val Asp Gln Leu Asp Tyr Trp Glu Ser Gly Glu Trp Val Ile Ile Asn
        195                 200                 205

Ala Val Gly Asn Tyr Asn Ser Lys Lys Tyr Glu Cys Cys Thr Glu Ile
    210                 215                 220

Tyr Pro Asp Ile Thr Tyr Ser Phe Ile Ile Arg Arg Leu Pro Leu Phe
225                 230                 235                 240

Tyr Thr Ile Asn Leu Ile Ile Pro Cys Leu Leu Ile Ser Cys Leu Thr
                245                 250                 255

Val Leu Val Phe Tyr Leu Pro Ser Glu Cys Gly Glu Lys Ile Thr Leu
            260                 265                 270

Cys Ile Ser Val Leu Leu Ser Leu Thr Val Phe Leu Leu Leu Ile Thr
        275                 280                 285

Glu Ile Ile Pro Ser Thr Ser Leu Val Ile Pro Leu Ile Gly Glu Tyr
    290                 295                 300

Leu Leu Phe Thr Met Ile Phe Val Thr Leu Ser Ile Ile Ile Thr Val
305                 310                 315                 320

Phe Val Leu Asn Val His His Arg Ser Pro Arg Thr His Thr Met Pro
                325                 330                 335

Asp Trp Val Arg Arg Val Phe Leu Asp Ile Val Pro Arg Leu Leu Phe
            340                 345                 350

Met Lys Arg Pro Ser Thr Val Lys Asp Asn Cys Lys Lys Leu Ile Glu
        355                 360                 365

Ser Met His Lys Leu Thr Asn Ser Pro Arg Leu Trp Ser Glu Thr Asp
    370                 375                 380

Met Glu Pro Asn Phe Thr Thr Ser Ser Ser Pro Ser Pro Gln Ser Asn
385                 390                 395                 400

Glu Pro Ser Pro Thr Ser Ser Phe Cys Ala His Leu Glu Glu Pro Ala
                405                 410                 415

Lys Pro Met Cys Lys Ser Pro Ser Gly Gln Tyr Ser Met Leu His Pro
            420                 425                 430

Glu Pro Pro Gln Val Thr Cys Ser Ser Pro Lys Pro Ser Cys His Pro
        435                 440                 445

Leu Ser Asp Thr Gln Thr Thr Ser Ile Ser Lys Gly Arg Ser Leu Ser
    450                 455                 460

Val Gln Gln Met Tyr Ser Pro Asn Lys Thr Glu Glu Gly Ser Ile Arg
465                 470                 475                 480

Cys Arg Ser Arg Ser Ile Gln Tyr Cys Tyr Leu Gln Glu Asp Ser Ser
                485                 490                 495

Gln Thr Asn Gly His Ser Ser Ala Ser Pro Ala Ser Gln Arg Cys His
            500                 505                 510

Leu Asn Glu Glu Gln Pro Gln His Lys Pro His Gln Cys Lys Cys Lys
        515                 520                 525

Cys Arg Lys Gly Glu Ala Ala Gly Thr Pro Thr Gln Gly Ser Lys Ser
    530                 535                 540

His Ser Asn Lys Gly Glu His Leu Val Leu Met Ser Pro Ala Leu Lys
545                 550                 555                 560

Leu Ala Val Glu Gly Val His Tyr Ile Ala Asp His Leu Arg Ala Glu
                565                 570                 575

Asp Ala Asp Phe Ser Val Lys Glu Asp Trp Lys Tyr Val Ala Met Val
            580                 585                 590

Ile Asp Arg Ile Phe Leu Trp Met Phe Ile Ile Val Cys Leu Leu Gly
        595                 600                 605

Thr Val Gly Leu Phe Leu Pro Pro Trp Leu Ala Gly Met Ile
    610                 615                 620

<210> 4

<211> 31
<212> DNA
<213> Künstliche Sequenz

<220>
<223> Beschreibung der künstlichen Sequenz: Primer

<400> 4
cacgtgccct ccgagctcat ctggcggccg g                                31

<210> 5
<211> 22
<212> DNA
<213> Künstliche Sequenz

<220>
<223> Beschreibung der künstlichen Sequenz: Primer

<400> 5
gtcatatgtc cacgagccga ac                                          22

<210> 6
<211> 1896
<212> DNA
<213> Künstliche Sequenz

<220>
<221> CDS
<222> (1)..(1893)

<220>
<223> Beschreibung der künstlichen Sequenz: Modifizierte
      alpha 4-Untereinheit des nikotinischen
      Acetylcholinrezeptors des Huhns

<400> 6
atg gga ttt ctc gtg tcg aag gga aac ctc ctc ctc ctg ctg tgt gcc   48
Met Gly Phe Leu Val Ser Lys Gly Asn Leu Leu Leu Leu Leu Cys Ala
  1               5                  10                  15

agc atc ttc ccc gct ttc ggc cac gtg gaa acg cga gcc cat gcg gag   96
Ser Ile Phe Pro Ala Phe Gly His Val Glu Thr Arg Ala His Ala Glu
             20                  25                  30

gag cgc ctc ctg aag aaa ctc ttc tcc ggg tat aac aag tgg tcc cgt   144
Glu Arg Leu Leu Lys Lys Leu Phe Ser Gly Tyr Asn Lys Trp Ser Arg

35                  40                  45

ccc gtc gcc aac att tcg gat gtg gtc ctg gtc cgc ttc ggc ttg tcc    192
Pro Val Ala Asn Ile Ser Asp Val Val Leu Val Arg Phe Gly Leu Ser
    50                  55                  60

ata gcc cag ctc atc gat gtt gat gag aag aac caa atg atg acc aca    240
Ile Ala Gln Leu Ile Asp Val Asp Glu Lys Asn Gln Met Met Thr Thr
65                  70                  75                  80

aat gtg tgg gtg aag cag gag tgg cac gac tac aag ctg cgc tgg gac    288
Asn Val Trp Val Lys Gln Glu Trp His Asp Tyr Lys Leu Arg Trp Asp
                85                  90                  95

ccc cag gag tat gaa aac gtc aca tcc atc cga atc ccc tca gag ctc    336
Pro Gln Glu Tyr Glu Asn Val Thr Ser Ile Arg Ile Pro Ser Glu Leu
            100                 105                 110

atc tgg agg ccg gac att gtc cta tac aac aat gct gat ggt gac ttt    384
Ile Trp Arg Pro Asp Ile Val Leu Tyr Asn Asn Ala Asp Gly Asp Phe
        115                 120                 125

gca gtc acc cac ctg acc aaa gcc cac ctc ttc tat gat ggg aga att    432
Ala Val Thr His Leu Thr Lys Ala His Leu Phe Tyr Asp Gly Arg Ile
    130                 135                 140

aaa tgg atg cca cct gcc atc tac aaa agc tcc tgc agc atc gat gtt    480
Lys Trp Met Pro Pro Ala Ile Tyr Lys Ser Ser Cys Ser Ile Asp Val
145                 150                 155                 160

acc ttc ttc ccc ttt gat cag caa aac tgt aaa atg aaa ttt ggc tct    528
Thr Phe Phe Pro Phe Asp Gln Gln Asn Cys Lys Met Lys Phe Gly Ser
                165                 170                 175

tgg aca tat gac aaa gct aag ata gac ttg gtg agc atg cat agc cat    576
Trp Thr Tyr Asp Lys Ala Lys Ile Asp Leu Val Ser Met His Ser His
            180                 185                 190

cgc ggg acc aac gtg gtg gag ctg ggc gtg gac caa ctg gac tac tgg    624
Arg Gly Thr Asn Val Val Glu Leu Gly Val Asp Gln Leu Asp Tyr Trp
        195                 200                 205

gaa agc ggg gag tgg gtc atc att aat gcc gtg ggc aat tac aac agc    672
Glu Ser Gly Glu Trp Val Ile Ile Asn Ala Val Gly Asn Tyr Asn Ser
    210                 215                 220

aag aaa tat gaa tgc tgc aca gag atc tac cct gat ata act tac tcc    720
Lys Lys Tyr Glu Cys Cys Thr Glu Ile Tyr Pro Asp Ile Thr Tyr Ser

225                 230                 235                 240

ttc att atc cgg agg ctg ccg ctg ttc tac aca atc aat ttg atc att     768
Phe Ile Ile Arg Arg Leu Pro Leu Phe Tyr Thr Ile Asn Leu Ile Ile
                245                 250                 255

ccc tgc ctg ctt atc tcc tgc ttg act gtc ctg gtc ttc tac cta ccc     816
Pro Cys Leu Leu Ile Ser Cys Leu Thr Val Leu Val Phe Tyr Leu Pro
            260                 265                 270

tct gag tgc gga gag aag ata acc ttg tgc atc tct gtg ctg cta tcc     864
Ser Glu Cys Gly Glu Lys Ile Thr Leu Cys Ile Ser Val Leu Leu Ser
        275                 280                 285

ctc acg gtg ttc ctg ctg ctc atc aca gag atc atc cct tct acc tcc     912
Leu Thr Val Phe Leu Leu Leu Ile Thr Glu Ile Ile Pro Ser Thr Ser
    290                 295                 300

ctg gtc atc ccc ctg ata gga gag tat ctg ctc ttc acc atg ata ttt     960
Leu Val Ile Pro Leu Ile Gly Glu Tyr Leu Leu Phe Thr Met Ile Phe
305                 310                 315                 320

gtc acc ttg tct atc atc atc act gtc ttt gtg ctc aac gta cac cac     1008
Val Thr Leu Ser Ile Ile Ile Thr Val Phe Val Leu Asn Val His His
                325                 330                 335

cgt tca cca cgt acc cac acg atg cct gac tgg gtg agg agg gtc ttc     1056
Arg Ser Pro Arg Thr His Thr Met Pro Asp Trp Val Arg Arg Val Phe
            340                 345                 350

ctt gac ata gtc cca cgt ctc ctc ttc atg aag cgg ccc tcc aca gtg     1104
Leu Asp Ile Val Pro Arg Leu Leu Phe Met Lys Arg Pro Ser Thr Val
        355                 360                 365

aaa gac aat tgc aag aag ctt att gaa tct atg cac aaa cta acc aac     1152
Lys Asp Asn Cys Lys Lys Leu Ile Glu Ser Met His Lys Leu Thr Asn
    370                 375                 380

tca cca agg ctt tgg tct gag acc gac atg gag ccc aac ttc act acc     1200
Ser Pro Arg Leu Trp Ser Glu Thr Asp Met Glu Pro Asn Phe Thr Thr
385                 390                 395                 400

tca tcc tcc ccc agc ccc cag agt aat gaa cct tca ccc aca tct tcc     1248
Ser Ser Ser Pro Ser Pro Gln Ser Asn Glu Pro Ser Pro Thr Ser Ser
                405                 410                 415

ttc tgt gcc cac ctt gag gag cca gcc aaa cct atg tgc aaa tcc cct     1296
Phe Cys Ala His Leu Glu Glu Pro Ala Lys Pro Met Cys Lys Ser Pro

            420                 425                 430

tct gga cag tac tca atg ctg cac cct gag ccc cca cag gtg acg tgt 1344
Ser Gly Gln Tyr Ser Met Leu His Pro Glu Pro Pro Gln Val Thr Cys
        435                 440                 445

tcc tct ccg aag ccc tcc tgc cac ccc ctg agt gac acc cag acc aca 1392
Ser Ser Pro Lys Pro Ser Cys His Pro Leu Ser Asp Thr Gln Thr Thr
    450                 455                 460

tct atc tca aaa ggc aga tcg ctc agt gtt cag cag atg tac agc ccc 1440
Ser Ile Ser Lys Gly Arg Ser Leu Ser Val Gln Gln Met Tyr Ser Pro
465                 470                 475                 480

aat aag aca gag gaa ggg agc atc cgc tgt agg tcc cga agc atc cag 1488
Asn Lys Thr Glu Glu Gly Ser Ile Arg Cys Arg Ser Arg Ser Ile Gln
                485                 490                 495

tac tgt tac ctg cag gag gac tct tcc cag acc aat ggc cac tct agt 1536
Tyr Cys Tyr Leu Gln Glu Asp Ser Ser Gln Thr Asn Gly His Ser Ser
            500                 505                 510

gcc tct cca gcg tcg cag cgc tgc cac ctc aat gaa gag cag ccc cag 1584
Ala Ser Pro Ala Ser Gln Arg Cys His Leu Asn Glu Glu Gln Pro Gln
        515                 520                 525

cac aag ccc cac cag tgc aag tgt aag tgc aga aag gga gag gca gct 1632
His Lys Pro His Gln Cys Lys Cys Lys Cys Arg Lys Gly Glu Ala Ala
    530                 535                 540

ggc aca ccg act caa gga agc aag agc cac agc aac aaa gga gaa cac 1680
Gly Thr Pro Thr Gln Gly Ser Lys Ser His Ser Asn Lys Gly Glu His
545                 550                 555                 560

ctc gtg ctg atg tcc cca gcc ctg aag ctg gcg gtg gaa ggg gtc cac 1728
Leu Val Leu Met Ser Pro Ala Leu Lys Leu Ala Val Glu Gly Val His
                565                 570                 575

tac att gca gac cac ctg cga gca gaa gat gca gat ttc tca gtg aag 1776
Tyr Ile Ala Asp His Leu Arg Ala Glu Asp Ala Asp Phe Ser Val Lys
            580                 585                 590

gaa gac tgg aag tac gta gca atg gtc att gac cgg atc ttt ctc tgg 1824
Glu Asp Trp Lys Tyr Val Ala Met Val Ile Asp Arg Ile Phe Leu Trp
        595                 600                 605

atg ttc atc atc gtg tgt ttg ctg ggg acc gtt ggg ctc ttc ctc ccg 1872
Met Phe Ile Ile Val Cys Leu Leu Gly Thr Val Gly Leu Phe Leu Pro

610                 615                 620

ccg tgg ctg gca gga atg atc taa                                    1896
Pro Trp Leu Ala Gly Met Ile
625                 630

<210> 7
<211> 631
<212> PRT
<213> Künstliche Sequenz
<223> Beschreibung der künstlichen Sequenz: Modifizierte
      alpha 4-Untereinheit des nikotinischen
      Acetylcholinrezeptors des Huhns

<400> 7
Met Gly Phe Leu Val Ser Lys Gly Asn Leu Leu Leu Leu Leu Cys Ala
  1               5                  10                  15

Ser Ile Phe Pro Ala Phe Gly His Val Glu Thr Arg Ala His Ala Glu
             20                  25                  30

Glu Arg Leu Leu Lys Lys Leu Phe Ser Gly Tyr Asn Lys Trp Ser Arg
         35                  40                  45

Pro Val Ala Asn Ile Ser Asp Val Val Leu Val Arg Phe Gly Leu Ser
     50                  55                  60

Ile Ala Gln Leu Ile Asp Val Asp Glu Lys Asn Gln Met Met Thr Thr
 65                  70                  75                  80

Asn Val Trp Val Lys Gln Glu Trp His Asp Tyr Lys Leu Arg Trp Asp
                 85                  90                  95

Pro Gln Glu Tyr Glu Asn Val Thr Ser Ile Arg Ile Pro Ser Glu Leu
            100                 105                 110

Ile Trp Arg Pro Asp Ile Val Leu Tyr Asn Asn Ala Asp Gly Asp Phe
        115                 120                 125

Ala Val Thr His Leu Thr Lys Ala His Leu Phe Tyr Asp Gly Arg Ile
    130                 135                 140

Lys Trp Met Pro Pro Ala Ile Tyr Lys Ser Ser Cys Ser Ile Asp Val
145                 150                 155                 160

Thr Phe Phe Pro Phe Asp Gln Gln Asn Cys Lys Met Lys Phe Gly Ser
                165                 170                 175

Trp Thr Tyr Asp Lys Ala Lys Ile Asp Leu Val Ser Met His Ser His
            180                 185                 190

Arg Gly Thr Asn Val Val Glu Leu Gly Val Asp Gln Leu Asp Tyr Trp
        195                 200                 205

Glu Ser Gly Glu Trp Val Ile Ile Asn Ala Val Gly Asn Tyr Asn Ser
    210                 215                 220

Lys Lys Tyr Glu Cys Cys Thr Glu Ile Tyr Pro Asp Ile Thr Tyr Ser
225                 230                 235                 240

Phe Ile Ile Arg Arg Leu Pro Leu Phe Tyr Thr Ile Asn Leu Ile Ile
                245                 250                 255

Pro Cys Leu Leu Ile Ser Cys Leu Thr Val Leu Val Phe Tyr Leu Pro
            260                 265                 270

Ser Glu Cys Gly Glu Lys Ile Thr Leu Cys Ile Ser Val Leu Leu Ser
        275                 280                 285

Leu Thr Val Phe Leu Leu Leu Ile Thr Glu Ile Ile Pro Ser Thr Ser
    290                 295                 300

Leu Val Ile Pro Leu Ile Gly Glu Tyr Leu Leu Phe Thr Met Ile Phe
305                 310                 315                 320

Val Thr Leu Ser Ile Ile Ile Thr Val Phe Val Leu Asn Val His His
                325                 330                 335

Arg Ser Pro Arg Thr His Thr Met Pro Asp Trp Val Arg Arg Val Phe
            340                 345                 350

Leu Asp Ile Val Pro Arg Leu Leu Phe Met Lys Arg Pro Ser Thr Val
        355                 360                 365

Lys Asp Asn Cys Lys Lys Leu Ile Glu Ser Met His Lys Leu Thr Asn
    370                 375                 380

Ser Pro Arg Leu Trp Ser Glu Thr Asp Met Glu Pro Asn Phe Thr Thr
385                 390                 395                 400

Ser Ser Ser Pro Ser Pro Gln Ser Asn Glu Pro Ser Pro Thr Ser Ser
                405                 410                 415

Phe Cys Ala His Leu Glu Glu Pro Ala Lys Pro Met Cys Lys Ser Pro
            420                 425                 430

Ser Gly Gln Tyr Ser Met Leu His Pro Glu Pro Pro Gln Val Thr Cys
        435                 440                 445

Ser Ser Pro Lys Pro Ser Cys His Pro Leu Ser Asp Thr Gln Thr Thr
    450                 455                 460

Ser Ile Ser Lys Gly Arg Ser Leu Ser Val Gln Gln Met Tyr Ser Pro
465                 470                 475                 480

Asn Lys Thr Glu Glu Gly Ser Ile Arg Cys Arg Ser Arg Ser Ile Gln
                485                 490                 495

Tyr Cys Tyr Leu Gln Glu Asp Ser Ser Gln Thr Asn Gly His Ser Ser
            500                 505                 510

Ala Ser Pro Ala Ser Gln Arg Cys His Leu Asn Glu Glu Gln Pro Gln
        515                 520                 525

His Lys Pro His Gln Cys Lys Cys Lys Cys Arg Lys Gly Glu Ala Ala
    530                 535                 540

Gly Thr Pro Thr Gln Gly Ser Lys Ser His Ser Asn Lys Gly Glu His
545                 550                 555                 560

Leu Val Leu Met Ser Pro Ala Leu Lys Leu Ala Val Glu Gly Val His
                565                 570                 575

Tyr Ile Ala Asp His Leu Arg Ala Glu Asp Ala Asp Phe Ser Val Lys
            580                 585                 590

Glu Asp Trp Lys Tyr Val Ala Met Val Ile Asp Arg Ile Phe Leu Trp
        595                 600                 605

Met Phe Ile Ile Val Cys Leu Leu Gly Thr Val Gly Leu Phe Leu Pro
    610                 615                 620

Pro Trp Leu Ala Gly Met Ile
625                 630

<210> 8
<211> 81
<212> DNA
<213> Künstliche Sequenz

<220>

<223> Beschreibung der künstlichen Sequenz: Primer

<400> 8
gctaagatag acttgagaca catcgatgag gctagaggaa ccaacgtggt agaactgggt 60
gtggaccaac tggactactg g 81

<210> 9
<211> 81
<212> DNA
<213> Künstliche Sequenz

<220>
<223> Beschreibung der künstlichen Sequenz: Primer

<400> 9
ccagtagtcc agttggtcca cacccagttc taccacgttg gttcctctag cctcatcgat 60
gtgtctcaag tctatcttag c 81

<210> 10
<211> 1869
<212> DNA
<213> Künstliche Sequenz

<220>
<221> CDS
<222> (1)..(1866)

<220>
<223> Beschreibung der künstlichen Sequenz: Modifizierte
      alpha 4-Untereinheit des nikotinischen
      Acetylcholinrezeptors des Huhns

<400> 10
atg gga ttt ctc gtg tcg aag gga aac ctc ctc ctc ctg ctg tgt gcc 48
Met Gly Phe Leu Val Ser Lys Gly Asn Leu Leu Leu Leu Leu Cys Ala
1               5                   10                  15

agc atc ttc ccc gct ttc ggc cac gtg gaa acg cga gcc cat gcg gag 96
Ser Ile Phe Pro Ala Phe Gly His Val Glu Thr Arg Ala His Ala Glu
            20                  25                  30

gag cgc ctc ctg aag aaa ctc ttc tcc ggg tat aac aag tgg tcc cgt 144
Glu Arg Leu Leu Lys Lys Leu Phe Ser Gly Tyr Asn Lys Trp Ser Arg
        35                  40                  45

ccc gtc gcc aac att tcg gat gtg gtc ctg gtc cgc ttc ggc ttg tcc 192

Pro Val Ala Asn Ile Ser Asp Val Val Leu Val Arg Phe Gly Leu Ser
    50                  55                  60

ata gcc cag ctc atc gat gtt gat gag aag aac caa atg atg acc aca 240
Ile Ala Gln Leu Ile Asp Val Asp Glu Lys Asn Gln Met Met Thr Thr
65                  70                  75                  80

aat gtg tgg gtg aag cag gag tgg cac gac tac aag ctg cgc tgg gac 288
Asn Val Trp Val Lys Gln Glu Trp His Asp Tyr Lys Leu Arg Trp Asp
                85                  90                  95

ccc cag gag tat gaa aac gtc aca tcc atc cga atc ccc tca gag ctc 336
Pro Gln Glu Tyr Glu Asn Val Thr Ser Ile Arg Ile Pro Ser Glu Leu
            100                 105                 110

atc tgg agg ccg gac att gtc cta tac aac aat gct gat ggt gac ttt 384
Ile Trp Arg Pro Asp Ile Val Leu Tyr Asn Asn Ala Asp Gly Asp Phe
        115                 120                 125

gca gtc acc cac ctg acc aaa gcc cac ctc ttc tat gat ggg aga att 432
Ala Val Thr His Leu Thr Lys Ala His Leu Phe Tyr Asp Gly Arg Ile
    130                 135                 140

aaa tgg atg cca cct gcc atc tac aaa agc tcc tgc agc atc gat gtt 480
Lys Trp Met Pro Pro Ala Ile Tyr Lys Ser Ser Cys Ser Ile Asp Val
145                 150                 155                 160

acc ttc ttc ccc ttt gat cag caa aac tgt aaa atg aaa ttt ggc tct 528
Thr Phe Phe Pro Phe Asp Gln Gln Asn Cys Lys Met Lys Phe Gly Ser
                165                 170                 175

tgg aca tat gac aaa gct aag ata gac ttg gtg agc atg cat agc cat 576
Trp Thr Tyr Asp Lys Ala Lys Ile Asp Leu Val Ser Met His Ser His
            180                 185                 190

gtc gac ctg tcc gag ttc tac acc tcc gtg gag tgg gac atc ctg gag 624
Val Asp Leu Ser Glu Phe Tyr Thr Ser Val Glu Trp Asp Ile Leu Glu
        195                 200                 205

gtg cca gcc gtc agg aac gag aag ttc tac acg tgc tgc gac gag ccc 672
Val Pro Ala Val Arg Asn Glu Lys Phe Tyr Thr Cys Cys Asp Glu Pro
    210                 215                 220

tac ctg gac ata acg ttt aac ttc att atc cgg agg ctg ccg ctg ttc 720
Tyr Leu Asp Ile Thr Phe Asn Phe Ile Ile Arg Arg Leu Pro Leu Phe
225                 230                 235                 240

tac aca atc aat ttg atc att ccc tgc ctg ctt atc tcc tgc ttg act 768

Tyr Thr Ile Asn Leu Ile Ile Pro Cys Leu Leu Ile Ser Cys Leu Thr
                245                 250                 255

gtc ctg gtc ttc tac cta ccc tct gag tgc gga gag aag ata acc ttg     816
Val Leu Val Phe Tyr Leu Pro Ser Glu Cys Gly Glu Lys Ile Thr Leu
            260                 265                 270

tgc atc tct gtg ctg cta tcc ctc acg gtg ttc ctg ctg ctc atc aca     864
Cys Ile Ser Val Leu Leu Ser Leu Thr Val Phe Leu Leu Leu Ile Thr
        275                 280                 285

gag atc atc cct tct acc tcc ctg gtc atc ccc ctg ata gga gag tat     912
Glu Ile Ile Pro Ser Thr Ser Leu Val Ile Pro Leu Ile Gly Glu Tyr
    290                 295                 300

ctg ctc ttc acc atg ata ttt gtc acc ttg tct atc atc atc act gtc     960
Leu Leu Phe Thr Met Ile Phe Val Thr Leu Ser Ile Ile Ile Thr Val
305                 310                 315                 320

ttt gtg ctc aac gta cac cac cgt tca cca cgt acc cac acg atg cct     1008
Phe Val Leu Asn Val His His Arg Ser Pro Arg Thr His Thr Met Pro
                325                 330                 335

gac tgg gtg agg agg gtc ttc ctt gac ata gtc cca cgt ctc ctc ttc     1056
Asp Trp Val Arg Arg Val Phe Leu Asp Ile Val Pro Arg Leu Leu Phe
            340                 345                 350

atg aag cgg ccc tcc aca gtg aaa gac aat tgc aag aag ctt att gaa     1104
Met Lys Arg Pro Ser Thr Val Lys Asp Asn Cys Lys Lys Leu Ile Glu
        355                 360                 365

tct atg cac aaa cta acc aac tca cca agg ctt tgg tct gag acc gac     1152
Ser Met His Lys Leu Thr Asn Ser Pro Arg Leu Trp Ser Glu Thr Asp
    370                 375                 380

atg gag ccc aac ttc act acc tca tcc tcc ccc agc ccc cag agt aat     1200
Met Glu Pro Asn Phe Thr Thr Ser Ser Ser Pro Ser Pro Gln Ser Asn
385                 390                 395                 400

gaa cct tca ccc aca tct tcc ttc tgt gcc cac ctt gag gag cca gcc     1248
Glu Pro Ser Pro Thr Ser Ser Phe Cys Ala His Leu Glu Glu Pro Ala
                405                 410                 415

aaa cct atg tgc aaa tcc cct tct gga cag tac tca atg ctg cac cct     1296
Lys Pro Met Cys Lys Ser Pro Ser Gly Gln Tyr Ser Met Leu His Pro
            420                 425                 430

gag ccc cca cag gtg acg tgt tcc tct ccg aag ccc tcc tgc cac ccc     1344

Glu Pro Pro Gln Val Thr Cys Ser Ser Pro Lys Pro Ser Cys His Pro
        435                 440                 445

ctg agt gac acc cag acc aca tct atc tca aaa ggc aga tcg ctc agt      1392
Leu Ser Asp Thr Gln Thr Thr Ser Ile Ser Lys Gly Arg Ser Leu Ser
    450                 455                 460

gtt cag cag atg tac agc ccc aat aag aca gag gaa ggg agc atc cgc      1440
Val Gln Gln Met Tyr Ser Pro Asn Lys Thr Glu Glu Gly Ser Ile Arg
465                 470                 475                 480

tgt agg tcc cga agc atc cag tac tgt tac ctg cag gag gac tct tcc      1488
Cys Arg Ser Arg Ser Ile Gln Tyr Cys Tyr Leu Gln Glu Asp Ser Ser
                485                 490                 495

cag acc aat ggc cac tct agt gcc tct cca gcg tcg cag cgc tgc cac      1536
Gln Thr Asn Gly His Ser Ser Ala Ser Pro Ala Ser Gln Arg Cys His
            500                 505                 510

ctc aat gaa gag cag ccc cag cac aag ccc cac cag tgc aag tgt aag      1584
Leu Asn Glu Glu Gln Pro Gln His Lys Pro His Gln Cys Lys Cys Lys
        515                 520                 525

tgc aga aag gga gag gca gct ggc aca ccg act caa gga agc aag agc      1632
Cys Arg Lys Gly Glu Ala Ala Gly Thr Pro Thr Gln Gly Ser Lys Ser
    530                 535                 540

cac agc aac aaa gga gaa cac ctc gtg ctg atg tcc cca gcc ctg aag      1680
His Ser Asn Lys Gly Glu His Leu Val Leu Met Ser Pro Ala Leu Lys
545                 550                 555                 560

ctg gcg gtg gaa ggg gtc cac tac att gca gac cac ctg cga gca gaa      1728
Leu Ala Val Glu Gly Val His Tyr Ile Ala Asp His Leu Arg Ala Glu
                565                 570                 575

gat gca gat ttc tca gtg aag gaa gac tgg aag tac gta gca atg gtc      1776
Asp Ala Asp Phe Ser Val Lys Glu Asp Trp Lys Tyr Val Ala Met Val
            580                 585                 590

att gac cgg atc ttt ctc tgg atg ttc atc atc gtg tgt ttg ctg ggg      1824
Ile Asp Arg Ile Phe Leu Trp Met Phe Ile Ile Val Cys Leu Leu Gly
        595                 600                 605

acc gtt ggg ctc ttc ctc ccg ccg tgg ctg gca gga atg atc taa          1869
Thr Val Gly Leu Phe Leu Pro Pro Trp Leu Ala Gly Met Ile
    610                 615                 620

<210> 11
<211> 622
<212> PRT
<213> Künstliche Sequenz
<223> Beschreibung der künstlichen Sequenz: Modifizierte
      alpha 4-Untereinheit des nikotinischen
      Acetylcholinrezeptors des Huhns

<400> 11
Met Gly Phe Leu Val Ser Lys Gly Asn Leu Leu Leu Leu Leu Cys Ala
1               5                   10                  15

Ser Ile Phe Pro Ala Phe Gly His Val Glu Thr Arg Ala His Ala Glu
            20                  25                  30

Glu Arg Leu Leu Lys Lys Leu Phe Ser Gly Tyr Asn Lys Trp Ser Arg
        35                  40                  45

Pro Val Ala Asn Ile Ser Asp Val Val Leu Val Arg Phe Gly Leu Ser
    50                  55                  60

Ile Ala Gln Leu Ile Asp Val Asp Glu Lys Asn Gln Met Met Thr Thr
65                  70                  75                  80

Asn Val Trp Val Lys Gln Glu Trp His Asp Tyr Lys Leu Arg Trp Asp
                85                  90                  95

Pro Gln Glu Tyr Glu Asn Val Thr Ser Ile Arg Ile Pro Ser Glu Leu
            100                 105                 110

Ile Trp Arg Pro Asp Ile Val Leu Tyr Asn Asn Ala Asp Gly Asp Phe
        115                 120                 125

Ala Val Thr His Leu Thr Lys Ala His Leu Phe Tyr Asp Gly Arg Ile
    130                 135                 140

Lys Trp Met Pro Pro Ala Ile Tyr Lys Ser Ser Cys Ser Ile Asp Val
145                 150                 155                 160

Thr Phe Phe Pro Phe Asp Gln Gln Asn Cys Lys Met Lys Phe Gly Ser
                165                 170                 175

Trp Thr Tyr Asp Lys Ala Lys Ile Asp Leu Val Ser Met His Ser His
            180                 185                 190

Val Asp Leu Ser Glu Phe Tyr Thr Ser Val Glu Trp Asp Ile Leu Glu
        195                 200                 205

Val Pro Ala Val Arg Asn Glu Lys Phe Tyr Thr Cys Cys Asp Glu Pro
    210                 215                 220

Tyr Leu Asp Ile Thr Phe Asn Phe Ile Ile Arg Arg Leu Pro Leu Phe
225                 230                 235                 240

Tyr Thr Ile Asn Leu Ile Ile Pro Cys Leu Leu Ile Ser Cys Leu Thr
                245                 250                 255

Val Leu Val Phe Tyr Leu Pro Ser Glu Cys Gly Glu Lys Ile Thr Leu
            260                 265                 270

Cys Ile Ser Val Leu Leu Ser Leu Thr Val Phe Leu Leu Leu Ile Thr
        275                 280                 285

Glu Ile Ile Pro Ser Thr Ser Leu Val Ile Pro Leu Ile Gly Glu Tyr
    290                 295                 300

Leu Leu Phe Thr Met Ile Phe Val Thr Leu Ser Ile Ile Ile Thr Val
305                 310                 315                 320

Phe Val Leu Asn Val His His Arg Ser Pro Arg Thr His Thr Met Pro
                325                 330                 335

Asp Trp Val Arg Arg Val Phe Leu Asp Ile Val Pro Arg Leu Leu Phe
            340                 345                 350

Met Lys Arg Pro Ser Thr Val Lys Asp Asn Cys Lys Lys Leu Ile Glu
        355                 360                 365

Ser Met His Lys Leu Thr Asn Ser Pro Arg Leu Trp Ser Glu Thr Asp
    370                 375                 380

Met Glu Pro Asn Phe Thr Thr Ser Ser Ser Pro Ser Pro Gln Ser Asn
385                 390                 395                 400

Glu Pro Ser Pro Thr Ser Ser Phe Cys Ala His Leu Glu Glu Pro Ala
                405                 410                 415

Lys Pro Met Cys Lys Ser Pro Ser Gly Gln Tyr Ser Met Leu His Pro
            420                 425                 430

Glu Pro Pro Gln Val Thr Cys Ser Ser Pro Lys Pro Ser Cys His Pro
        435                 440                 445

Leu Ser Asp Thr Gln Thr Thr Ser Ile Ser Lys Gly Arg Ser Leu Ser
    450                 455                 460

Val Gln Gln Met Tyr Ser Pro Asn Lys Thr Glu Glu Gly Ser Ile Arg
465                 470                 475                 480

Cys Arg Ser Arg Ser Ile Gln Tyr Cys Tyr Leu Gln Glu Asp Ser Ser
                485                 490                 495

Gln Thr Asn Gly His Ser Ser Ala Ser Pro Ala Ser Gln Arg Cys His
            500                 505                 510

Leu Asn Glu Glu Gln Pro Gln His Lys Pro His Gln Cys Lys Cys Lys
        515                 520                 525

Cys Arg Lys Gly Glu Ala Ala Gly Thr Pro Thr Gln Gly Ser Lys Ser
    530                 535                 540

His Ser Asn Lys Gly Glu His Leu Val Leu Met Ser Pro Ala Leu Lys
545                 550                 555                 560

Leu Ala Val Glu Gly Val His Tyr Ile Ala Asp His Leu Arg Ala Glu
                565                 570                 575

Asp Ala Asp Phe Ser Val Lys Glu Asp Trp Lys Tyr Val Ala Met Val
            580                 585                 590

Ile Asp Arg Ile Phe Leu Trp Met Phe Ile Ile Val Cys Leu Leu Gly
        595                 600                 605

Thr Val Gly Leu Phe Leu Pro Pro Trp Leu Ala Gly Met Ile
    610                 615                 620

<210> 12
<211> 79
<212> DNA
<213> Künstliche Sequenz

<220>
<223> Beschreibung der künstlichen Sequenz: Primer

<400> 12
caacagcaag aaatatgaat gctgcgacga gccctacctt gatataactt tcaacttcat 60
tatccggagg ctgccgctg                                              79

<210> 13
<211> 79
<212> DNA

<213> Künstliche Sequenz

<220>
<223> Beschreibung der künstlichen Sequenz: Primer

<400> 13
cagcggcagc ctccggataa tgaagttgaa agttatatca aggtagggct cgtcgcagca 60
ttcatatttc ttgctgttg 79

<210> 14
<211> 30
<212> DNA
<213> Künstliche Sequenz

<220>
<223> Beschreibung der künstlichen Sequenz: Primer

<400> 14
gaacaaaagc tggaggtcca ccgcggtggc 30

<210> 15
<211> 30
<212> DNA
<213> Künstliche Sequenz

<220>
<223> Beschreibung der künstlichen Sequenz: Primer

<400> 15
gccaccgcgg tggacctcca gcttttgttc 30

<210> 16
<211> 75
<212> DNA
<213> Künstliche Sequenz

<220>
<223> Beschreibung der künstlichen Sequenz: Primer

<400> 16
gcggggagtg ggtcatctta gaagtcccgg ccgttcgcaa cgaaaagttt tatacatgct 60
gcgacgagcc ctacc 75

<210> 17

<211> 75
<212> DNA
<213> Künstliche Sequenz

<220>
<223> Beschreibung der künstlichen Sequenz: Primer

<400> 17
ggtagggctc gtcgcagcat gtataaaact tttcgttgcg aacggccggg acttcaatga 60
tgacccactc cccgc 75